(19) Europäisches Patentamt
European Patent Office
Office européen des brevets

(11) EP 2 196 189 B1

(12) FASCICULE DE BREVET EUROPEEN

(45) Date de publication et mention de la délivrance du brevet:
**06.03.2019 Bulletin 2019/10**

(51) Int Cl.:
***A61K 8/49*** *(2006.01)* ***A61K 8/41*** *(2006.01)*
***A61Q 17/04*** *(2006.01)*

(21) Numéro de dépôt: **10154126.6**

(22) Date de dépôt: **14.11.2002**

# (54) Compositions cosmétiques antisolaires à base d'un mélange synergique de filtres et utilisations

Kosmetische Sonnenschutzmittel auf Basis einer synergistischen Mischung von Filtern und deren Verwendungen

Cosmetic sunscreen compositions based on synergistic filter mixture and their use

(84) Etats contractants désignés:
**AT BE BG CH CY CZ DE DK EE ES FI FR GB GR IE IT LI LU MC NL PT SE SK TR**

(30) Priorité: **07.12.2001 FR 0115860**

(43) Date de publication de la demande:
**16.06.2010 Bulletin 2010/24**

(62) Numéro(s) de document de la (des) demande(s) initiale(s) en application de l'article 76 CBE:
**02292840.2 / 1 317 918**

(73) Titulaire: **L'Oréal**
**75008 Paris (FR)**

(72) Inventeur: **Candau, Didier**
**91570, BIEVRES (FR)**

(74) Mandataire: **Casalonga, Axel**
**Casalonga & Partners**
**Bayerstrasse 71-73**
**80335 München (DE)**

(56) Documents cités:
**EP-A- 0 893 119**
**EP-A- 1 046 391**
**EP-A- 1 133 980**
**EP-A- 1 219 287**
**EP-A2- 1 290 999**
**EP-A2- 1 310 235**
**WO-A-95/22959**
**WO-A1-03/039507**
**GB-A- 2 303 549**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention concerne de nouvelles compositions cosmétiques ou dermatologiques à usage topique, en particulier pour la photoprotection de la peau et/ou des cheveux, caractérisées par le fait qu'elles comprennent, dans un support cosmétiquement acceptable, au moins :

(a) à titre de premier filtre, un filtre UV organique insoluble, de taille de particule allant de 10 nm à 5 μm et choisi parmi les filtres UV organiques du type oxalanilide ; du type triazine ; du type amide vinylique ; du type cinnamamide ; du type comportant un ou plusieurs groupements benzazole et/ou benzofuranne, benzothiophène ou du type indole ; du type aryl vinylène cétone ; du type dérivé de phénylène bis-benzoxazinone du type dérivé amide, sulfonamide ou carbamate d'acrylonitrile et
(b) un dérivé de 2-hydroxybenzophénone amino-substitué particulier, à titre de second filtre.

L'association de ces deux filtres conduit à l'obtention d'un effet de synergie au niveau des facteurs de protection solaire UV-$A_{PPD}$ conférés.
L'invention concerne également leurs applications à la protection de la peau et des cheveux contre les effets du rayonnement ultraviolet.
On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ; ce rayonnement UV-B doit donc être filtré.
On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.
De nombreuses compositions cosmétiques destinées à la photoprotection (UV-A et/ou UV-B) de la peau ont été proposées à ce jour.
L'efficacité des compositions anti-solaires s'exprime généralement par le facteur de protection solaire (FPS) s'exprimant mathématiquement par le rapport de la dose de rayonnement UV nécessaire pour atteindre le seuil érythématogène avec le filtre UV avec la dose de rayonnement UV nécessaire pour atteindre le seuil érythématogène sans filtre UV. Ce facteur concerne donc l'efficacité de la protection vis à vis de l'érythème dont le spectre d'action biologique est centré dans l'UVB et par conséquent, rend compte de la protection vis à vis de ce rayonnement UV-B.
Compte tenu des effets des UV-A sur la peau et du développement de nombreuses compositions contenant des associations de filtres capables d'absorber le rayonnement UV-B et/ou UV-A; il s'est développé des méthodes spécifiques d'évaluation de la protection contre le rayonnement UV-A.
Pour caractériser la protection vis à vis des UV-A, la méthode PPD (Persistent Pigment Darkening), qui mesure la couleur de la peau observée 2 à 4 heures après une exposition de la peau aux UV-A, est particulièrement recommandée et utilisée. Cette méthode est adoptée depuis 1996 par la Japanese Cosmetic Industry Association (JCIA) en tant que procédure officielle de test pour l'étiquetage UV-A des produits et est fréquemment utilisée par les laboratoires de tests en Europe et aux Etats-Unis; (Japan Cosmetic Industry Association Technical Bulletin. Measurement Standards for UVA protection efficacy. Issued November 21, 1995 and efective of January 1, 1996).
Le facteur de protection solaire $UVA_{PPD}$ (FP $UVA_{PPD}$) s'exprime mathématiquement par le rapport de la dose de rayonnement UV-A nécessaire pour atteindre le seuil de pigmentation avec le filtre UV ($MPPD_p$) avec la dose de rayonnement UV-A nécessaire pour atteindre le seuil de pigmentation sans filtre UV ($MPPD_{np}$).

$$\text{FP UVA}_{\text{PPD}} = \frac{\text{MPPDp}}{\text{MPPDnp}}$$

Ces compositions antisolaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction des facteurs de protection solaire recherchés.
On connaît dans les demandes de brevet EP-A-1046391 et DE10012408 des compositions antisolaires à base de dérivés de 2-hydroxybenzophénone aminosubstitués. Le document EP1133980 décrit des compositions comprenant

des composés de triazine non micronisés et des dérivés d'aminohydroxybenzophénone pour la protection solaire.

Or, à la suite d'importantes recherches menées dans le domaine de la photoprotection évoqué ci-dessus, la Demanderesse a découvert, de façon inattendue et surprenante, que la combinaison, dans des proportions comprises dans des limites bien déterminées, de deux filtres solaires particuliers et déjà connus en soi dans l'état de l'art, permettait, du fait d'un effet de synergie remarquable, d'obtenir des compositions antisolaires présentant des facteurs de protection solaire UV-$A_{PPD}$ nettement améliorés, et en tous cas largement supérieurs à ceux qui peuvent être obtenus soit avec l'un ou l'autre des filtres utilisé seul.

Cette découverte est à la base de la présente invention.

Ainsi, conformément à l'un des objets de la présente invention, il est maintenant proposé de nouvelles compositions cosmétiques à usage topique, en particulier pour la photoprotection de la peau et/ou des cheveux, caractérisées par le fait qu'elles comprennent, dans un support cosmétiquement acceptable :

(a) à titre de premier filtre, au moins un filtre UV organique insoluble, de taille de particule allant de 10 nm à 5 $\mu$m et choisi parmi les filtres UV organiques du type oxalanilide ; du type triazine ; du type amide vinylique ; du type cinnamamide ; du type comportant un ou plusieurs groupements benzazole et/ou benzofuranne, benzothiophène ou du type indole ; du type aryl vinylène cétone ; du type dérivé de phénylène bis-benzoxazinone du type dérivé amide, sulfonamide ou carbamate d'acrylonitrile et

(b) au moins un dérivé de 2-hydroxybenzophénone amino-substitué de formule (I) que l'on définira plus loin, à titre de second filtre.

La présente invention a également pour objet l'utilisation de telles compositions pour la fabrication de compositions cosmétiques destinées à la protection de la peau et/ou des cheveux contre le rayonnement ultraviolet, en particulier le rayonnement solaire.

La présente invention a également pour objet l'utilisation d'un dérivé de 2-hydroxybenzophénone amino-substitué de formule (I) que l'on définira plus loin pour la fabrication de compositions cosmétiques ou dermatologiques destinées à la protection de la peau et/ou des cheveux contre le rayonnement ultraviolet, en particulier le rayonnement solaire comprenant au moins un filtre UV organique, insoluble de taille de particule allant de 10 nm à 5 $\mu$m, dans le but de produire un effet synergique au niveau des facteurs de protection solaire UV-$A_{PPD}$ conférés.

Par filtre UV insoluble, au sens de la présente invention, on entend tout filtre UV organique ou minéral ayant une solubilité dans l'eau inférieure à 0,1 % en poids et une solubilité inférieure à 1 % en poids dans la plupart des solvants organiques comme l'huile de paraffine, les benzoates d'alcools gras et les triglycérides d'acides gras, par exemple le Miglyol® 812 commercialisé par la société DYNAMIT NOBEL. Cette solubilité, définie à 70 °C comme la quantité de produit en solution dans le solvant à l'équilibre avec un excès de solide en suspension, peut facilement être évaluée au laboratoire.

D'autres caractéristiques, aspects et avantages de la présente invention apparaîtront à la lecture de la description détaillée qui va suivre.

De façon générale, le filtre UV insoluble et le dérivé de 2-hydroxybenzophénone amino-substitué sont présents dans lesdites compositions dans une proportion produisant une activité synergique au niveau des facteurs de protection solaire UV-$A_{PPD}$ conférés.

Les filtres UV organiques insolubles selon l'invention ont une taille moyenne des particules qui varie de 10 à 5$\mu$m et plus préférentiellement de 10 nm à 2 $\mu$m et plus particulièrement de 20 nm à 2 $\mu$m.

Les filtres organiques insolubles selon l'invention peuvent être amenés sous la forme particulaire souhaitée par tout moyen ad-hoc tel que notamment broyage à sec ou en milieu solvant, tamisage, atomisation, micronisation, pulvérisation.

Les filtres organiques insolubles selon l'invention sous forme micronisée peuvent en particulier être obtenus par un procédé de broyage d'un filtre UV organique insoluble sous forme de particules de taille grossière en présence d'un tensio-actif approprié permettant d'améliorer la dispersion des particules ainsi obtenues dans les formulations cosmétiques.

Un exemple de procédé de micronisation de filtres organiques insolubles est décrit dans les demandes GB-A-2 303 549 et EP-A-893119 faisant partie intégrante de la description. L'appareil de broyage utilisé selon ces documents peut être un broyeur à jet, à billes, à vibration ou à marteau et de préférence un broyeur à haute vitesse d'agitation ou un broyeur à impact et plus particulièrement un broyeur à billes rotatives, un broyeur vibrant, à broyeur à tube ou un broyeur à tige.

Selon ce procédé particulier, on utilise à titre de tensio-actifs pour le broyage desdits filtres, les alkylpolyglucosides de structure $C_nH_{2n+1}O(C_6H_{10}O_5)_xH$ dans laquelle n est un entier de 8 à 16 et x est le degré moyen de polymérisation de l'unité $(C_6H_{10}O_5)$ et varie de 1,4 à 1,6. Ils peuvent être choisis parmi des esters en $C_1$-$C_{12}$ d'un composé de structure $C_nH_{2n+1}O(C_6H_{10}O_5)_xH$ et plus précisément un ester obtenu par réaction d'un acide carboxylique en $C_1$-$C_{12}$ tel que l'acide formique, acétique, propionique, butyrique, sulfosuccinique, citrique ou tartrique avec une ou

plusieurs fonctions OH libres sur l'unité glucoside ($C_6H_{10}O_5$). Lesdits tensio-actifs sont utilisés en général à une concentration de allant de 1 à 50% en poids et plus préférentiellement de 5 à 40% en poids par rapport au filtre insoluble dans sa forme micronisée.

Les filtres UV organiques insolubles conformes à l'invention sont choisis parmi les filtres UV organiques du type oxalanilide, du type triazine ; du type amide vinylique ; du type cinnamamide ; du type comportant un ou plusieurs groupements benzazole et/ou benzofuranne, benzothiophène ou du type indole ; du type aryl vinylène cétone ; du type dérivé de phénylène bis-benzoxazinone, du type dérivé amide, sulfonamide ou carbamate d'acrylonitrile.

Au sens où on l'utilise dans la présente invention, le terme benzazole englobe à la fois les benzothiazoles, benzoxazoles et benzimidazoles.

Parmi les filtres UV du type oxalanilide conformes à l'invention, on peut citer ceux répondant à la structure :

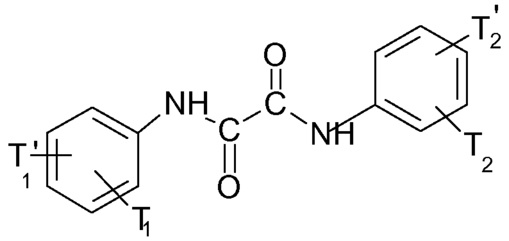

(1)

dans laquelle $T_1$ , $T'_1$, $T_2$ et $T'_2$ désignent, identiques et différents, un atome d'hydrogène, un radical alkyle en $C_1$-$C_8$ ou un radical alcoxy en $C_1$-$C_8$. Ces composés sont décrits dans la demande de brevet WO95/22959.

A titre d'exemples , on peut citer les produits commerciaux TINUVIN 315 et TINUVIN 312 vendus par la Société CIBA-GEIGY et respectivement de structure :

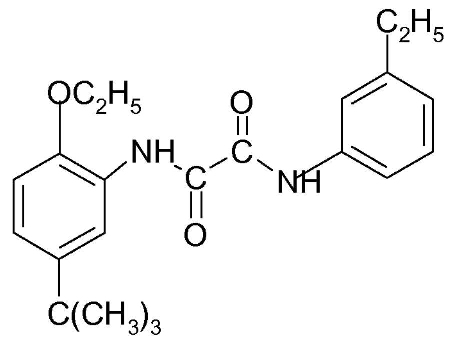

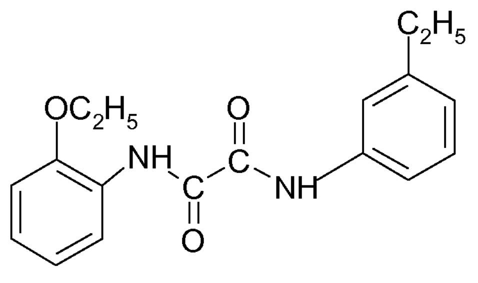

Parmi les filtres UV insolubles du type triazine conformes à l'invention, on peut également mentionner ceux répondant à la formule (2) suivante :

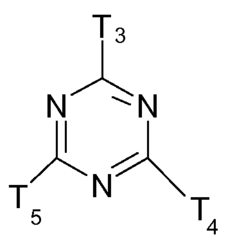

(2)

dans laquelle $T_3$, $T_4$, $T_5$, indépendamment, sont phenyle, phenoxy, pyrrolo, dans lesquels les phenyle, phenoxy, pyrrolo non substitués ou substitués par un, deux ou trois substituants choisis parmi OH, $C_1$-$C_{18}$alkyle ou C1-C18alkoxy, $C_1$-$C_{18}$carboxyalkyle, $C_5$-$C_8$cycloalkyle, un groupe méthylbenzylidènecamphre, un groupe -$(CH=CH)_n(CO)$-$OT_6$ , avec $T_6$ désignant $C_1$-$C_{18}$alkyle ou cinnamyle et n vaut 0 ou 1.

Ces composés sont décrits dans WO 97/03642, GB 2286774, EP-743309, WO 98/22447, GB 2319523 (faisant partie intégrante du contenu de la description).

Parmi les filtres UV du type triazine conformes à l'invention, on peut également mentionner les dérivés insolubles de s-triazine portant des groupements benzalmalonates et/ou phenylcyanoacrylates tels que ceux décrits dans la demande EP-A-0790243 (faisant partie intégrante du contenu de la description).

Parmi ces filtres UV insolubles du type triazine, on citera plus particulièrement les composés suivants :

- la 2,4,6-tris(4'-amino benzalmalonate de diéthyle)-s-triazine,
- la 2,4,6-tris(4'-amino benzalmalonate de diisopropyle)-s-triazine,
- la 2,4,6-tris(4'-amino benzalmalonate de diméthyle)-s-triazine,
- la 2,4,6-tris($\alpha$-cyano-4-aminocinnamate d'éthyle)-s-triazine.

Parmi les filtres UV du type triazine conformes à l'invention, on peut encore mentionner les dérivés insolubles de s-triazine portant des groupements benzotriazoles et/ou benzothiazoles tels que ceux décrits dans la demande WO98/25922 (faisant partie intégrante du contenu de la description).

Parmi ces composés, on peut citer plus particulièrement :

- la 2,4,6-tris[(3'-benzotriazol-2-yl-2'-hydroxy-5'-methyl) phenylamino]-s-triazine,
- 2,4,6-tris[(3'-benzotriazol-2-yl-2'-hydroxy-5'-ter-octyl) phénylamino]-s-triazine.

Parmi les filtres organiques insolubles du type amide vinylique, on peut citer par exemple les composés de formule suivante qui sont décrits dans la demande WO95/22959 (faisant partie intégrante du contenu de la description) :

$$T_{12}\text{-}(Y)r\text{-}C(=O)\text{-}C(T_{13})=C(T_{14})\text{-}N(T_{15})(T_{16}) \qquad (5)$$

dans laquelle $T_{12}$ est un radical alkyle en $C_1$-$C_{18}$, de préférence en $C_1$-$C_5$ ou un groupe phényle éventuellement substitué par un, deux ou trois radicaux choisis parmi OH, alkyle en $C_1$-$C_{18}$, alcoxy en $C_1$-$C_8$, ou un groupe -C(=O)-$OT_{17}$ où $T_{17}$ est un alkyle en $C_1$-$C_{18}$ ; $T_{13}$, $T_{14}$, $T_{15}$ et $T_{16}$ identiques ou différents désignent un radical alkyle en $C_1$-$C_{18}$, de préférence en $C_1$-$C_5$ ou un atome d'hydrogène ; Y est N ou O et r vaut 0 ou 1.

Parmi ces composés, on citera plus particulièrement :

- la 4-octylamino-3-pentèn-2-one ;
- l'éthyl-3-octylamino-2-buténoate ;
- la 3-octylamino-1-phényl-2-butèn-1-one
- la 3-dodecylamino-1-phenyl-2-buten-1-one.

Parmi les filtres organiques insolubles du type cinnamamide conformes à l'invention, on peut citer également les composés tels que décrits dans la demande WO95/22959 (faisant partie intégrante du contenu de la description) et répondant à la structure suivante :

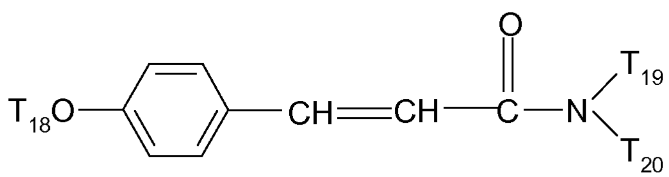

(6)

dans laquelle $OT_{18}$ est un radical hydroxy ou alcoxy en $C_1$-$C_4$, de préférence méthoxy ou éthoxy ; $T_{19}$ est hydrogène, alkyle en $C_1$-$C_4$, de préférence méthyle ou éthyle ; $T_{20}$ est un groupe -(CONH)s-phényle où s vaut 0 ou 1 et le groupe phényle peut être substitué par un, deux ou trois groupes choisis parmi OH, alkyle en $C_1$-$C_{18}$, alcoxy en $C_1$-$C_8$, ou un groupe -C(=O)-$OT_{21}$ où $T_{21}$ est un alkyle en $C_1$-$C_{18}$ et plus préférentiellement $T_{21}$ est un groupe phényle, 4-méthoxyphényle ou phénylaminocarbonyle.

On peut également citer les dimères cinnamamides tels que ceux décrits dans le brevet US 5888481 comme par exemple le composé de structure :

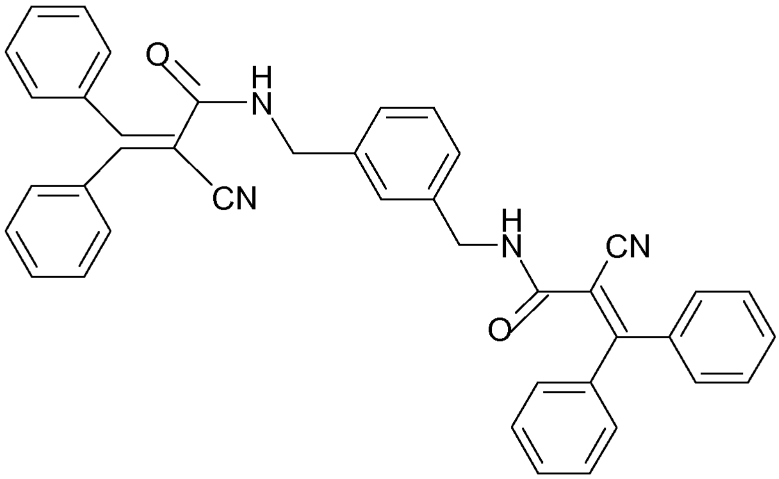

Parmi les filtres organiques insolubles du type benzazole, on peut citer ceux répondant à l'une des formules suivantes :

(7) 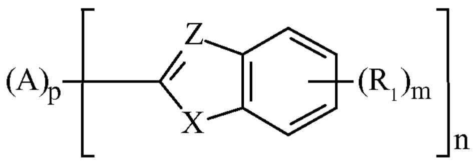

(8) 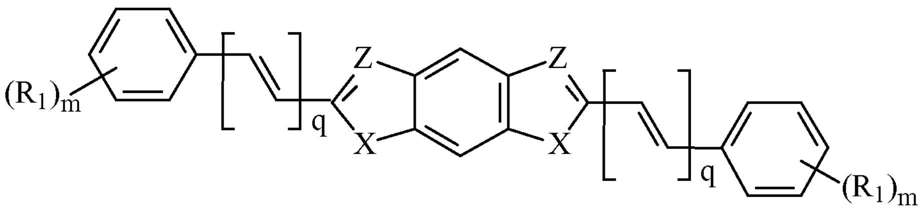

(9) 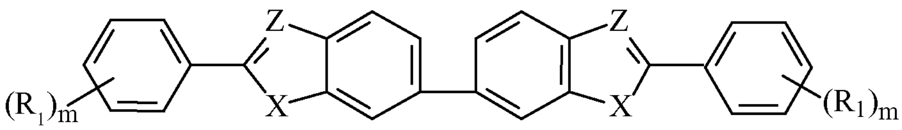

dans lesquelles chacun des symboles X représente indépendamment un atome d'oxygène ou de soufre ou un groupe $NR_2$,

chacun des symboles Z représente indépendamment un atome d'azote ou un groupe CH,

chacun des symboles $R_1$ représente indépendamment un groupe OH, un atome d'halogène, un groupe alkyle en $C_{1-8}$, linéaire ou ramifié, contenant éventuellement un atome de silicium, ou un groupe alcoxy en $C_{1-8}$, linéaire ou ramifié,

chacun des nombres m vaut indépendamment 0, 1 ou 2,

n représente un nombre entier compris entre 1 et 4 inclus,

p est égal à 0 ou 1,

chacun des nombres q est égal indépendamment à 0 ou 1,

chacun des symboles $R_2$ représente indépendamment un atome d'hydrogène, un groupe benzyle ou alkyle en $C_{1-8}$, linéaire ou ramifié, contenant éventuellement un atome de silicium,

A représente un radical de valence n choisi parmi ceux de formules :

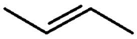

(a)

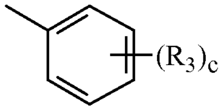

(b)

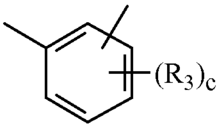

(c)

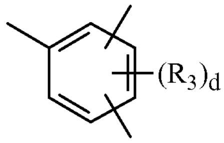

(d)

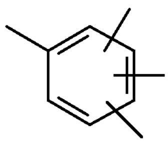

(e)

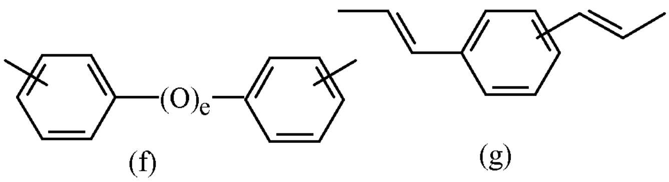

(f)

(g)

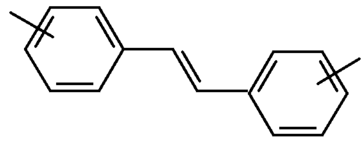

(h)

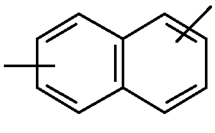

(i)

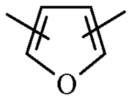

(j)

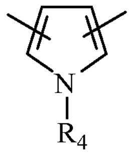

(k)

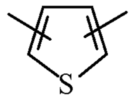

(l)

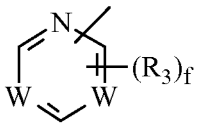

(m)

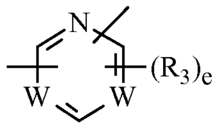

(n)

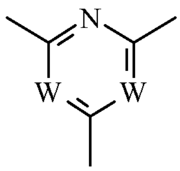

(o)

dans lesquelles W désigne N ou CH ; chacun des symboles $R_3$ représente indépendamment un atome d'halogène ou un groupe alkyle ou alcoxy en $C_{1-4}$, linéaire ou ramifié, ou hydroxy,
$R_4$ représente un atome d'hydrogène ou un groupe alkyle en $C_{1-4}$, linéaire ou ramifié, c = 0-4, d = 0 - 3, e = 0 ou 1, et f = 0 - 2.

Ces composés sont notamment décrits dans les brevets DE 676 103 et CH 350 763, le brevet US 5 501 850, le brevet US 5 961 960, la demande de brevet EP0669323, le brevet US 5 518 713, le brevet US 2 463 264, l'article du J. Am. Chem. Soc., 79, 5706 - 5708, 1957, l'article du J. Am. Chem. Soc., 82, 609 - 611, 1960, la demande de brevet EP0921126, la demande de brevet EP712855.

A titre d'exemples de composés préférés de formule (7) de la famille des 2-arylbenzazoles, on peut mentionner le 2-benzoxazol-2-yl-4-méthylphénol, le 2-(1H-benzimidazol-2-yl)-4-méthoxyphénol ou le 2-benzothiazol-2-ylphénol, ces composés pouvant être préparés par exemple selon les procédés décrits dans le brevet CH 350 763.

A titre d'exemples de composés préférés de formule (7) de la famille des benzimidazolylbenzazoles, on citera le 2,2'-bis-benzimidazole, le 5,5',6,6'-tétraméthyl-2,2'-bis-benzimidazole, le 5,5'-diméthyl-2,2'-bis-benzimidazole, le 6-méthoxy-2,2'-bis-benzimidazole, le 2-(1H-benzimidazol-2-yl)-benzothiazole, le 2-(1H-benzimidazol-2-yl)-benzoxazole et le N,N'-diméthyl-2,2'-bis-benzimidazole, ces composés pouvant être préparés selon les modes opératoires décrits dans les brevets US 5 961 960 et US 2 463 264.

A titre d'exemples de composés préférés de formule (7) de la famille des phénylène-benzazoles, on citera le 1,4-phénylène-bis-(2-benzoxazolyle), le 1,4-phénylène-bis-(2-benzimidazolyle), le 1,3-phénylène-bis-(2-benzoxazolyle), le 1,2-phénylène-bis-(2-benzoxazolyle), le 1,2-phénylène-bis-(benzimidazolyle), le 1,4-phénylène-bis-(N-2-éthylhexyl-2-benzimidazolyle) et le 1,4-phénylène-bis-(N-triméthylsilylméthyl-2-benzimidazolyle), ces composés pouvant être préparés selon les modes opératoires décrits dans le brevet US 2 463 264 et dans les publications J. Am.Chem. Soc., 82, 609 (1960) et J. Am. Chem. Soc., 79, 5706 - 5708 (1957).

A titre d'exemples de composés préférés de formule (7) de la famille des benzofuranyl-benzoxazoles, on citera le 2-(2-benzofuranyl)-benzoxazole, le 2-(benzofuranyl)-5-méthylbenzoxazole et le 2-(3-méthyl-2-benzofuranyle)-benzoxazole, ces composés pouvant être préparés selon les modes opératoires décrits dans le brevet US 5 518 713.

Comme composés préférés de formule (8), on peut citer par exemple le 2,6-diphényl-1,7-dihydro-benzo[1,2-d;4,5-d']-di-imidazole correspondant à la formule

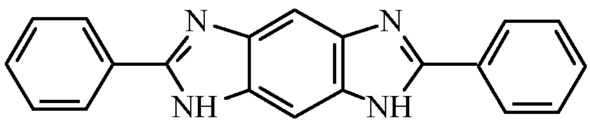

ou le 2,6-distyryl-1,7-dihydro-benzo[1,2-d ; 4,5-d']-di-imidazole ou encore le 2,6-di(p-tert-butylstyryl)-1,7-dihydrobenzo[1,2-d ; 4,5-d']-di-imidazole, qui peuvent être préparés selon la demande EP 0 669 323.

Comme composé préféré de formule (9), on peut citer le 5,5'-bis-[(phényl-2)-benzimidazole] de formule :

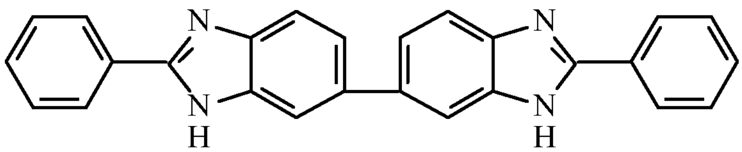

dont la préparation est décrite dans J. Chim. Phys., 64, 1602 (1967).

Parmi ces composés organiques insolubles filtrant le rayonnement UV, on préfère tout particulièrement le 2-(1H-benzimidazol-2-yl)benzoxazole, le 6-méthoxy-2,2'-bis-benzimidazole, le 2-(1H-benzimidazol-2-yl)-benzothiazole, le 1,4-phénylène-bis-(2-benzoxazolyle), le 1,4-phénylène-bis-(2-benzimidazolyle), le 1,3-phénylène-bis-(2-benzoxazolyle), le 1,2-phénylène-bis-(2-benzoxazolyle), le 1,2-phénylène-bis-(2-benzimidazolyle) et le 1,4-phénylène-bis-(N-triméthylsilylméthyl-2-benzimidazolyle).

Parmi les filtres organiques insolubles du type aryl vinylène cétone, on peut citer ceux correspondant à l'une des formules (10) et (11) suivantes :

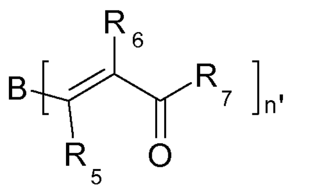

(10)

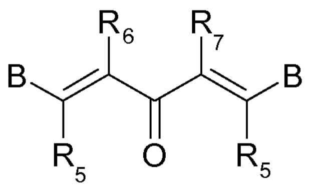

(11)

dans lesquelles :

n' = 1 ou 2,

B, dans la formule (I) lorsque n'=1 ou dans la formule (11), est un radical aryle choisi parmi les formules (a') à (d') suivantes, ou dans la formule (10) lorsque n'=2, est un radical choisi parmi les formules (e') à (h') suivantes :

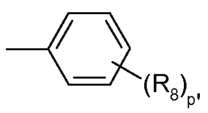

(a')

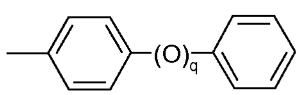

(b')

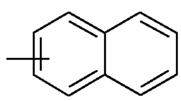

(c')

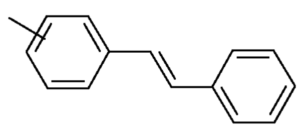

(d')

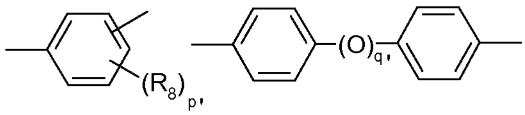 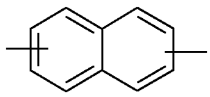 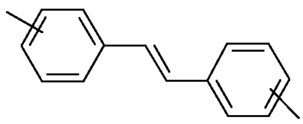

(e') (f') (g') (h')

dans lesquelles :

chacun des symboles $R_8$ représente indépendamment un groupe OH, un atome d'halogène, un groupe alkyle en $C_{1-6}$, linéaire ou ramifié et contenant éventuellement un atome de silicium, un groupe alcoxy en $C_{1-6}$, linéaire ou ramifié et contenant éventuellement un atome de silicium, un groupe alcoxycarbonyle en $C_{1-5}$, linéaire ou ramifié, ou un groupe alkylsulfonamide en $C_{1-6}$, linéaire ou ramifié et contenant éventuellement un atome de silicium ou une fonction aminoacide,

p' représente un nombre entier compris entre 0 et 4 inclus,

q' représente 0 ou 1,

$R_5$ représente l'hydrogène ou un groupe OH,

$R_6$ représente l'hydrogène, un groupe alkyle en $C_{1-6}$, linéaire ou ramifié et contenant éventuellement un atome de silicium, un groupe cyano, un groupe alkylsulfonyle en $C_{1-6}$, un groupe phénylsulfonyle,

$R_7$ représente un groupe alkyle en $C_{1-6}$, linéaire ou ramifié et contenant éventuellement un atome de silicium ou un groupe phényle pouvant former un bicycle et éventuellement substitué par un ou deux radicaux $R_4$,

ou $R_6$ et $R_7$ forment ensemble un reste hydrocarboné en $C_{2-10}$ monocyclique, bicyclique ou tricylique, éventuellement interrompu par un ou des atomes d'azote, de soufre et d'oxygène et pouvant contenir un autre carbonyle, et éventuellement substitué par un groupe alkylsulfonamide en $C_1$-$C_8$, linéaire ou ramifié, et contenant éventuellement un atome de silicium ou une fonction aminoacide ; à condition que lorsque n'=1, $R_6$ et $R_7$ ne forment pas un noyau camphre.

A titre d'exemples de composés de formule (10) dans laquelle n' =1, insolubles, filtrant le rayonnement UV, ayant une taille moyenne de particules comprise entre 10 nm et 5 nm, on peut mentionner les familles suivantes :

- les composés du type styryl cétone tels que décrits dans la demande JP 04 134 042 telle que la 1-(3,4-diméthoxy-phényl)-4,4-diméthyl-pent-1-èn-3-one :

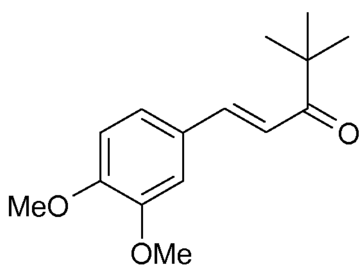

- les composés du type benzylidène cinéole tels que ceux décrits dans l'article de E. Mariani et al, 16th IFSCC Congress, New York (1990)) tel que la 1,3,3-triméthyl-5-(4-méthoxy-benzylidène)-2-oxa-bicyclo[2.2.2]octan-6-one :

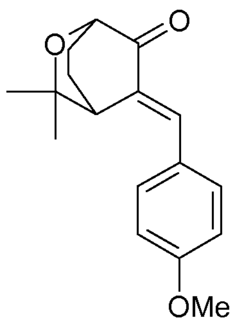

- les composés du type benzylidène chromanone tels que ceux décrits dans la demande JP 04 134 043 comme la 3-(4-méthoxy-benzylidène)-2,3,4a,8a-tétrahydro-chromèn-4-one :

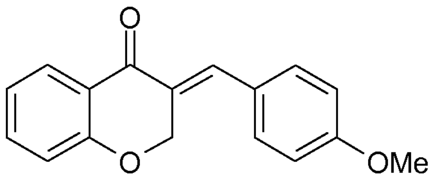

- les composés du type benzylidène thiochromanone tels que ceux décrits dans la demande JP 04 134 043 comme la 3-(4-méthoxy-benzylidène)-2,3,4a,8a-tétrahydro-chromèn-4-thione :

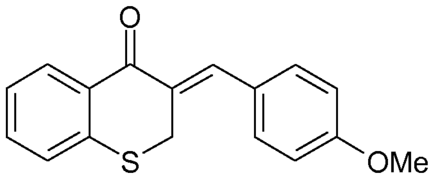

- les composés du type benzylidène quinuclidinone tels que ceux décrits dans la demande EP 0 576 974 comme la 4-méthoxy benzylidène-1-azabicyclo[2.2.2]octan-3-one :

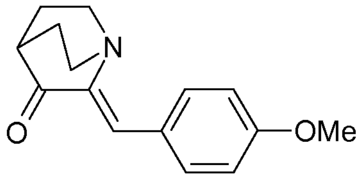

- les composés du type benzylidène cycloalcanone tels que ceux décrits dans la demande FR 2 395 023 comme les 2-(4-méthoxy-benzylidène)-cyclopentanone et 2-(4-méthoxy-benzyl-idène)-cyclohexanone :

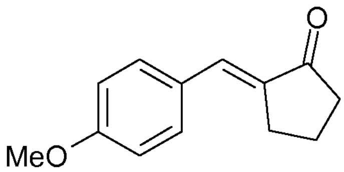

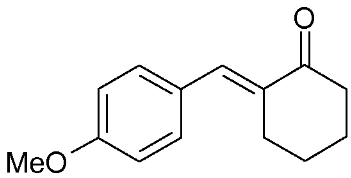

- les composés du type benzylidène hydantoïne tels que ceux décrits dans la demande JP 01 158 090 comme la 5-(3,4-diméthoxy-benzylidène)-imidazolidine-2,4-dione :

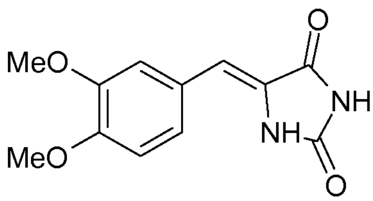

- les composés du type benzylidène indanone tels que ceux décrits dans la demande JP 04 134 043 comme la 2-(4-méthoxy-benzylidène)-indan-1-one :

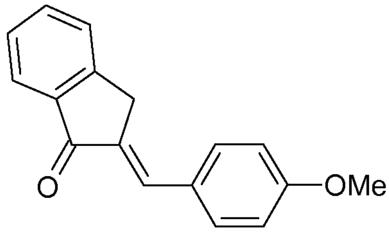

- les composés du type benzylidène tétralone tels que ceux décrits dans la demande JP 04 134 043 comme la 2-(4-méthoxy-benzylidène)-3,4-dihydro-2H-naphthalen-1-one :

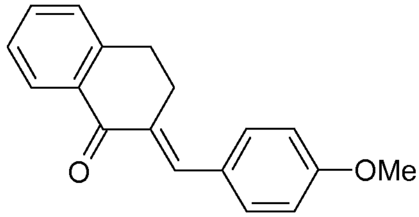

- les composés du type benzylidène furanone tels que ceux décrits dans la demande EP 0 390 683 comme la 4-(4-méthoxy-benzylidène)-2,2,5,5-tétraméthyl-dihydro-furan-3-one :

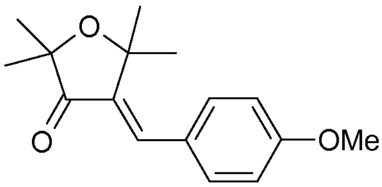

- les composés du type benzylidène benzofuranone tels que ceux décrits dans la demande JP 04 134 041 comme la 2-benzylidène-benzofuran-3-one :

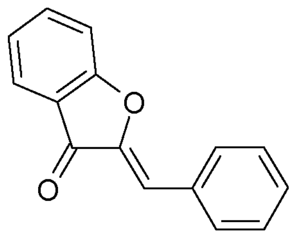

- les composés du type benzylidène indanedione telle que la 2-(3,5-di-tert-butyl-4-hydroxy-benzylidène)-indan-1,3-dione :

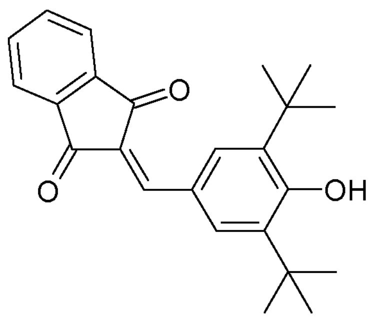

- les composés du type benzylidène benzothiofuranone comme ceux décrits dans la demande JP 04,134,043) comme la 2-benzylidène-benzo[b]thiophen-3-one :

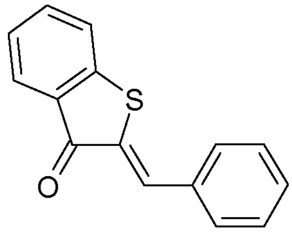

- les composés du type benzylidène barbiturique tels que la 5-(4-méthoxy-benzylidène)-1,3-diméthyl-pyrimidine-2,4,6-trione :

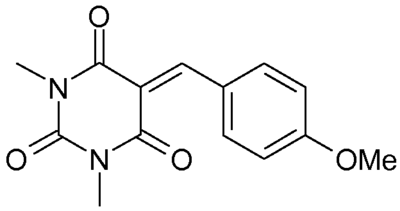

- les composés du type benzylidène pyrazolone comme la 4-(4-méthoxy-benzylidène)-5-méthyl-2-phényl-2,4-dihydro-pyrazol-3-one :

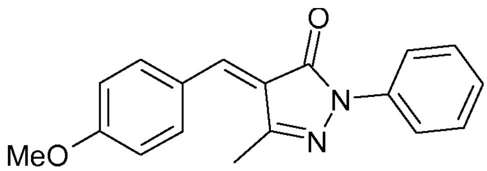

- les composés du type benzylidène imidazolone tels que la 5-(4-méthoxy-benzylidène)-2-phényl-3,5-dihydro-imidazol-4-one :

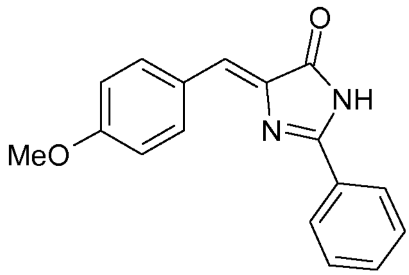

- les composés du type chalcone tels que la 1-(2-hydroxy-4-méthoxy-phényl)-3-phényl-propènone :

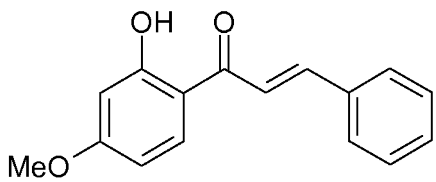

- les composés benzylidène one tels que décrits dans le document FR 2 506 156 comme la 3-hydroxy-1-(2-hydroxy-4-méthoxy-phényl)-3-phényl-propènone :

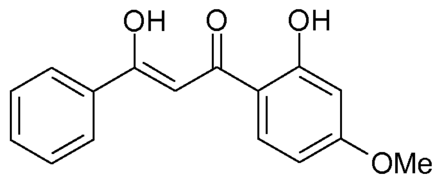

A titre d'exemples de composés de formule (10) dans laquelle n'=2 insolubles, filtrant le rayonnement UV, ayant une taille moyenne de particules comprise entre 10 nm et 5 μm, on peut mentionner les familles suivantes :

- les composés du type phénylène bis méthylidène-nor-camphre tels que décrits dans le document EP 0 693 471 comme la 1,4-phénylène-bis-{3-méthylidène-bicyclo[2.2.1]heptan-2-one} :

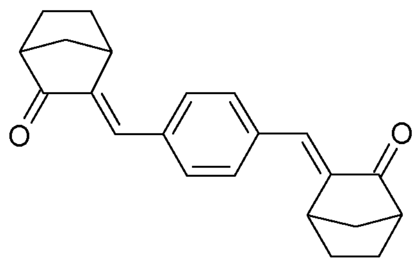

- les composés du type phénylène bis méthylidène camphre tels que décrits dans le document FR 2 528 420 comme la 1,4-phénylène-bis-{3-méthylidène-1,7,7-triméthyl-bicyclo [2.2.1]heptan-2-one} :

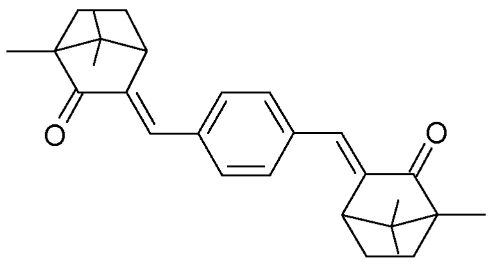

ou la 1,3-phénylène-bis-{3-méthylidène-1,7,7-triméthyl-bicyclo [2.2.1]heptan-2-one} :

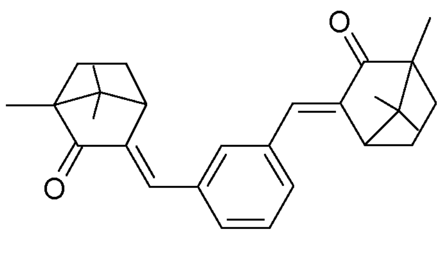

- les composés du type phénylène bis méthylidène camphre sulfonamide tels que ceux décrits dans le document FR2 529 887 comme le 1,4-phénylène-bis-3,3'-méthylidène camphre-10,10'-sulfonamide d'éthyle ou de 2-éthylhexyle :

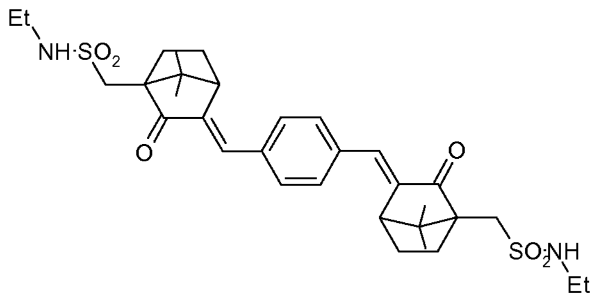

ou

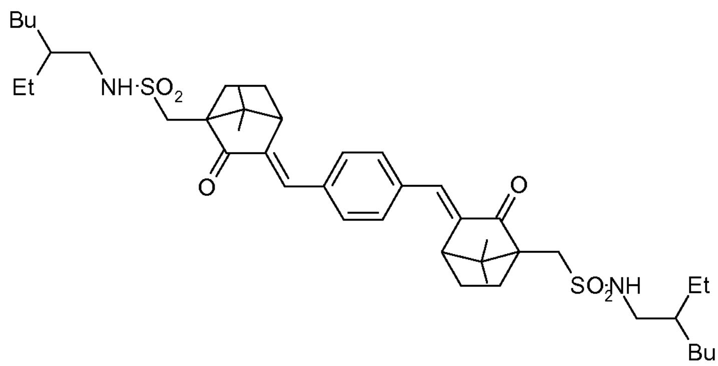

- les composés du type phénylène bis méthylidène cinéole tels que décrits dans l'article E. Mariani et al, 16th IFSCC Congress, New York (1990) comme la 1,4-phénylène-bis-{5-méthylidène-3,3-diméthyl-2-oxa-bicyclo[2.2.2]octan-6-one} :

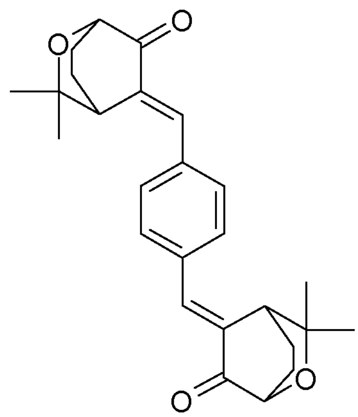

- les composés du type phénylène bis méthylidène cétotricyclodécane tels que décrits dans la demande EP 0 694 521 commel la 1,4-phénylène-bis-(octahydro-4,7-méthano-6-indèn-5-one) :

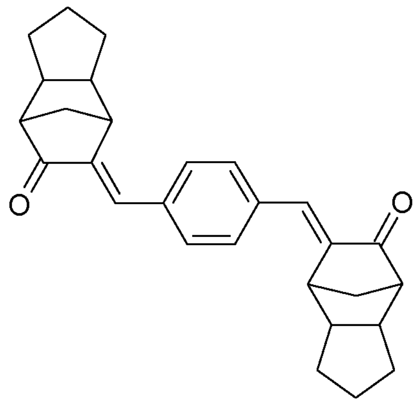

- les composés du type phénylène bis alkylène cétone tels que ceux décrits dans la demande JP 04 134 041 comme la 1,4-phénylène-bis-(4,4-diméthyl-pent-1-èn-3-one) :

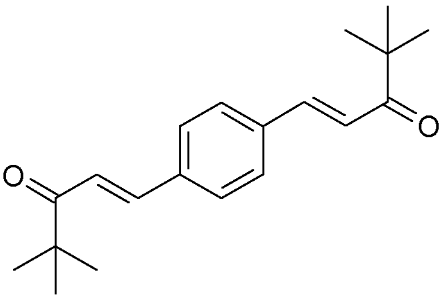

- les composés du type phénylène bis méthylidène furanone tels que décrits dans la demande FR 2 638 354 comme la 1,4-phénylène-bis-(4-méthylidène-2,2,5,5-tétraméthl-dihdrofuran-3-one :

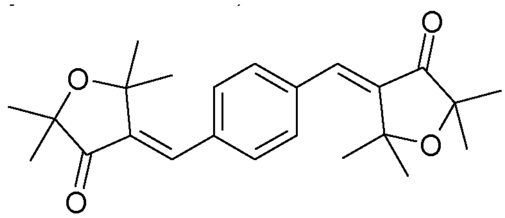

- les composés du type phénylène bis méthylidène quinuclidinone tels que ceuc décrits dans la demande EP 0 714 880 comme la 1,4-phénylène-bis-{2-méthylidène-1-aza-bicyclo [2.2.2]octan-3-one} :

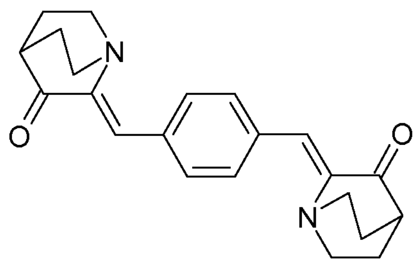

A titre de composés de formule (11), on peut mentionner les familles suivantes :

- les composés du type bis benzylidène cycloalcanone tels que la 2,5-dibenzylidène-cyclopentanone :

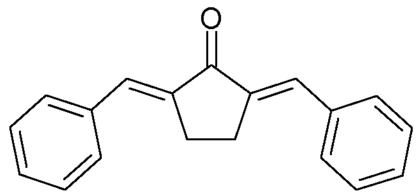

- les composés du type gamma pyrone tels que décrits dans le document JP 04 290 882 comme la 2,6-bis-(3,4-diméthoxy-phényl)-pyran-4-one :

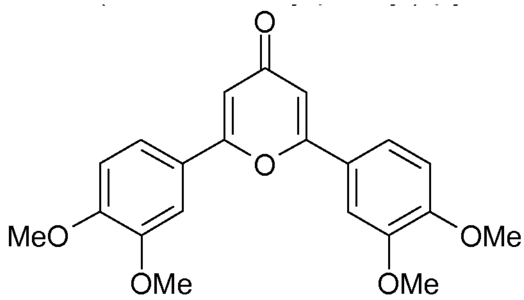

Parmi ces composés organiques insolubles filtrant le rayonnement UV du type aryl vinylène cétone, on préfère tout particulièrement les composés de formule (10) dans laquelle n'=2.

Parmi les filtres organiques insolubles du type phénylène bis-benzoxazinone, on peut citer ceux répondant à la formule (12) suivante :

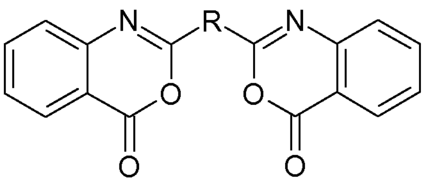

(12)

avec R représentant un reste aromatique divalent choisi parmi les formules (e) à (h) suivantes :

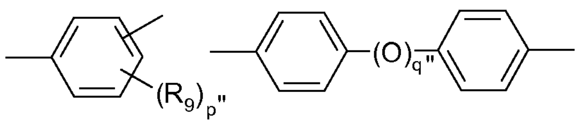

(e") (f")

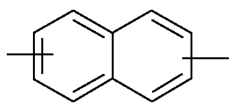

(g")

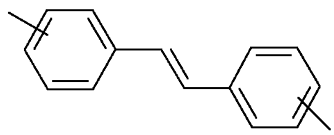

(h")

dans lesquelles :

chacun des symboles $R_9$ représente indépendamment un groupe OH, un atome d'halogène, un groupe alkyle en $C_{1-6}$, linéaire ou ramifié et contenant éventuellement un atome de silicium, un groupe alcoxy en $C_{1-6}$, linéaire ou ramifié et contenant éventuellement un atome de silicium, un groupe alcoxycarbonyle en $C_{1-5}$, linéaire ou ramifié, ou un groupe alkylsulfonamide en $C_{1-6}$, linéaire ou ramifié et contenant éventuellement un atome de silicium ou une fonction aminoacide,
p" représente un nombre entier compris entre 0 et 4 inclus,
q" représente 0 ou 1,

A titre d'exemples de composés de formule (12), insolubles, filtrant le rayonnement UV, ayant une taille moyenne de particules comprise entre 10 nm et 5 nm, on peut mentionner les dérivés suivants :

- le 2,2'-p-phénylène bis(3,1-benzoxazin-4-one), produit commercial CYASORB UV-3638 de la société CYTEC,

- le 2,2'-(4,4'-biphénylène) bis (3,1-benzoxazin-4-one),
- le 2,2'-(2,6-naphthylène) bis (3,1-benzoxazin-4-one).

Parmi les filtres organiques insolubles du type dérivé amide, sulfonamide ou carbamate d'acrylonitrile, on peut citer ceux répondant à la formule (13) suivante :

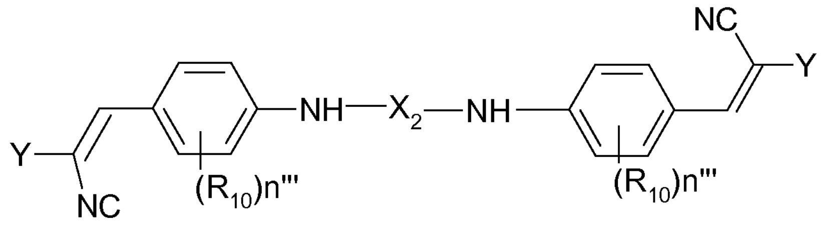

(13)

dans laquelle :

$R_{10}$ représente un groupe alkyle en $C_{1-8}$, linéaire ou ramifié,
n''' vaut 0, 1 ou 2,
$X_2$ représente un radical divalent de formule -(C=O)-$R_{11}$-(C=O)- , -$SO_2$-$R_{11}$-$SO_2$- ou -(C=O)-O-$R_{11}$-O-(C=O)-,
Y représente un radical -(C=O)-$R_{12}$ ou -$SO_2R_{13}$,
$R_{11}$ représente une simple liaison ou un radical divalent alkylène en $C_1$-$C_{30}$ ou alcénylène en $C_3$-$C_{30}$, linéaire ou ramifié, pouvant porter un ou plusieurs substituants hydroxyle et pouvant contenir, dans la chaîne carbonée, un ou plusieurs hétéroatomes choisis parmi les atomes d'oxygène, d'azote et de silicium,.
$R_{12}$ représente un radical -$OR_{14}$ ou -$NHR_{14}$,
$R_{13}$ représente un radical alkyle en $C_1$-$C_{30}$, linéaire ou ramifié, ou un noyau phényle non substitué ou substitué par des radicaux alkyle ou alcoxy en $C_1$-$C_4$,
$R_{14}$ représente un radical alkyle en $C_1$-$C_{30}$ ou alcényle en $C_3$-$C_{30}$, linéaire ou ramifié, pouvant porter un ou plusieurs substituants hydroxyle et pouvant contenir, dans la chaîne carbonée, un ou plusieurs hétéroatomes choisis parmi les atomes d'oxygène, d'azote et de silicium.

Bien que dans la formule (13) ci-dessus, seuls soient représentés les isomères dans lesquelles le substituant cyano est en position *cis* par rapport au substituant para-amino-phényle, cette formule doit être comprise comme englobant également les isomères *trans* correspondants. ; pour chacune des deux double liaisons et de façon indépendante, les substituants cyano et para-amino-phényle peuvent être en configuration *cis* ou *trans* l'un par rapport à l'autre.

A titre d'exemple, on peut citer le dimère de 2-cyano-3-[4-(acétylamino)phényl]-acrylate de 2-éthylhexyle de formule :

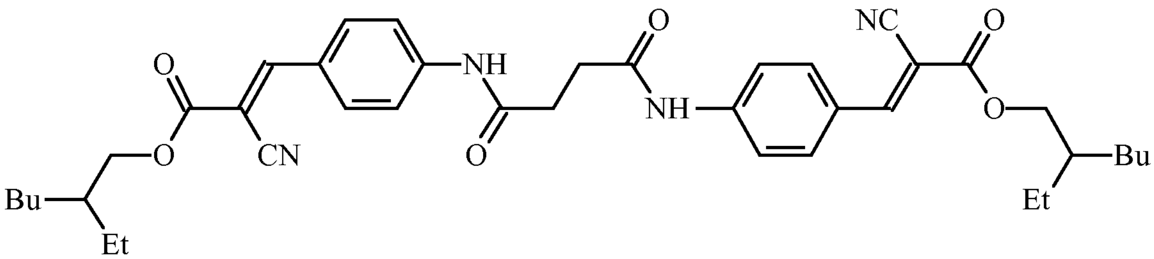

Une autre famille particulière de filtres organiques insolubles conformes à l'invention sont les sels de métaux polyvalents (par exemple $Ca^{2+}$ , $Zn^{2+}$ , $Mg^{2+}$, $Ba^{2+}$, $Al^{3+}$ ou $Zr^{4+}$ ) de filtres organiques sulfoniques ou carboxyliques tels que les sels de métaux polyvalents de dérivés sulfonés de benzylidène camphre tels que ceux décrits dans la demande FR-A 2 639 347 ; les sels de métaux polyvalents de dérivés sulfonés de benzimidazole tels que ceux décrits dans la demande EP-A-893119; les sels de métaux polyvalents de dérivés d'acide cinnamique tels que ceux décrits dans la demande JP-87 166 517.

On peut également citer les complexes de métaux ou d'ammonium ou d'ammonium substitué de filtres organiques UV-A et/ou UV-B tels que décrits dans les demandes de brevet WO93/10753, WO93/11095 et WO95/05150.

Le ou les filtres UV insolubles de l'invention sont présents à une concentration totale allant de préférence de 1 et 10 % en poids environ et plus particulièrement de 2 à 8% en poids par rapport au poids total de la composition.

Les dérivés de 2-hydroxybenzophénone aminosubstitués conformes à l'invention répondent à la formule (I) suivante :

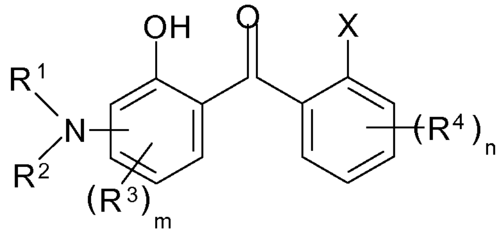

(I)

dans laquelle :

$R^1$ et $R^2$, identiques ou différents, désignent un atome d'hydrogène, un radical alkyle en $C_1$-$C_{20}$, un radical alcènyle en $C_2$-$C_{10}$, un radical cycloalkyle en $C_3$-$C_{10}$, un radical cycloalcènyle en $C_3$-$C_{10}$ ;
$R^1$ et $R^2$ peuvent également former avec l'atome d'azote avec lequel ils sont liés un cycle à 5 ou 6 chaînons ;
$R^3$ et $R^4$, identiques ou différents, désignent un radical alkyle en $C_1$-$C_{20}$, un radical alcènyle en $C_2$-$C_{10}$, un radical cycloalkyle en $C_3$-$C_{10}$, un radical cycloalcènyle en $C_3$-$C_{10}$, un radical alcoxy en C1-C12, un radical ($C_1$-$C_{20}$)alcoxy-carbonyle, un radical alkylamino en $C_1$-$C_{12}$, un radical dialkylamino en $C_1$-$C_{12}$, un radical aryle ou un hétéroaryle éventuellement substitué, un substituant hydrosolubilisant choisi parmi un groupe carboxylate, un groupe sulfonate ou un reste ammonium ; X désigne un atome d'hydrogène, un groupe $COOR^5$ ou $CONR^6R^7$;
$R^5$, $R^6$ et $R^7$, identiques ou différents, désignent un atome d'hydrogène, un radical alkyle en $C_1$-$C_{20}$, un radical alcènyle en $C_2$-$C_{10}$, un radical cycloalkyle en $C_3$-$C_{10}$, un radical cycloalcènyle en $C_3$-$C_{10}$, un groupe $-(YO)_o-Z$ ou un groupe aryle ;
Y désigne $-(CH_2)_2-$, $-(CH_2)_3-$ $-(CH_2)_4-$, $-CH-(CH_3)-CH_2-$ ;
Z représente $-CH_2-CH_3$, $-CH_2CH_2CH_3$, $-CH_2-CH_2-CH_2-CH_3$, $-CH(CH_3)-CH_3$ ;
m est un entier variant de 0 à3 ;
n est un entier variant de 0 à 3 ;
o est un entier variant de 1 à 2.

Comme radicaux alkyle en $C_1$-$C_{20}$, on peut citer par exemple : méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 2,2-diméthylpropyle, 1-éthylpropyle, n-hexyle, 1,1-diméthylpropyle, 1,2-diméthylpropyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,2,2-triméthylpropyle, 1-éthyl-1-méthylpropyle, 1-éthyl-2-méthylpropyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle ou n-eicosyle.

Comme groupes alcènyle en $C_2$-$C_{10}$, on peut citer par exemple : vinyle, n-propènyle, isopropènyle, 1-butènyle, 2-butènyle, 1-pentènyle, 2-pentènyle, 2-méthyl-1-butènyle, 2-méthyl-2-butènyle, 3-méthyl-1-butènyle, 1-hexènyle, 2-hexènyle, 1-heptènyle, 2-heptènyle, 1-octènyle, 2-octènyle.

Comme radicaux alcoxy en $C_1$-$C_{12}$, on peut citer : méthoxy, éthoxy, n-propoxy, n-butoxy, n-pentoxy, 1-méthylpropoxy, 3-méthylbutoxy, 2,2-diméthylpropoxy, 1-méthyl- 1-éthylpropoxy, octoxy, 2-méthylpropoxy, 1,1-diméthylpropoxy, hexoxy, heptoxy, 2-éthylhexoxy.

Comme radicaux cycloalkyles en $C_3$-$C_{10}$, on peut citer par exemple : cyclopropyle, cyclobutyle, cyclopentyle, cyclohexyle, cycloheptyle, 1 -méthylcyclopropyle, 1 -éthylcyclopropyle, 1-propylcyclopropyle, 1-butylcyclopropyle, 1-pentylcyclopropyle, 1-méthyl-1-butylcyclopropyle, 1,2-diméthylcyclypropyle, 1-méthyl-2-éthylcyclopropyle, cyclooctyle, cyclononyle ou cyclodécyle.

Comme radicaux cycloalcènyles en $C_3$-$C_{10}$ ayant une ou plusieurs doubles liaisons, on peut citer : cyclopropènyle, cyclobutènyle, cyclopentènyle, cyclopentadiènyle, cyclohexènyle, 1,3-cyclohexadiènyle, 1,4-cyclohexadiènyle, cycloheptènyle, cycloheptatriènyle, cyclooctènyle, 1,5-cyclooctadiènyle, cyclooctatétraènyle, cyclononènyle ou cyclodécènyle.

Les radicaux cycloalkyles ou cycloalcényles peuvent comporter un ou plusieurs substituants (de préférence de 1 à 3) chois par exemple parmi halogène comme chlore, fluor ou brome ; cyano ; nitro ; amino ; $C_1$-$C_4$-alkylamino ; $C_1$-$C_4$ dialkylamino ; $C_1$-$C_4$alkyle ; $C_1$-$C_4$-alcoxy ; hydroxy ; ils peuvent également comporter de 1 à 3 hétéroatomes comme souffre, oxygène ou azote dont les valences libres peuvent être occupées par un hydrogène ou un radical alkyle en $C_1$-$C_4$.

Les groupes aryles sont de préférence choisis parmi les cycles phényle ou naphtyle, lesquels pouvant comporter

un ou plusieurs substituants (de préférence de 1 à 3) chois par exemple parmi halogène comme chlore, fluor ou brome ; cyano ; nitro ; amino ; $C_1$-$C_4$-alkylamino ; $C_1$-$C_4$dialkylamino ; $C_1$-$C_4$alkyle ; $C_1$-$C_4$-alcoxy ; hydroxy. On préfère plus particulièrement phényle, méthoxyphényle et naphtyle.

Les groupes hétéroaryles comportent en général un ou plusieurs hétéroatomes choisis parmi souffre, oxygène ou azote.

Les groupes hydrosolubilisants sont par exemple des groupes carboxylates, sulfonates et plus particulièrement leurs sels avec des cations physiologiquement acceptables comme les sels de métaux alcalins ou les sels de trialkylammonium comme les sels de tri(hydroxyalkyl)ammonium ou de 2-méthylpropan-1-ol-2-ammonium. On peut également citer les groupes ammonium comme les alkylammoniums et leurs formes salifiées avec des anions physiologiquement acceptables.

Comme exemples de cycle à 5 ou 6 chaînons formé par les radicaux $R^1$ et $R^2$ avec l'atome d'azote, on peut citer en particulier pyrrolidine ou pipéridine.

Les groupes amino peuvent être fixés sur le noyau benzénique en position ortho, méta ou para par rapport au radical carbonyle et plus préférentiellement en para.

**[0074]** Une famille de composés de formule (I) préférentiels comprend ceux choisis parmi ceux de formule (Ia) suivante :

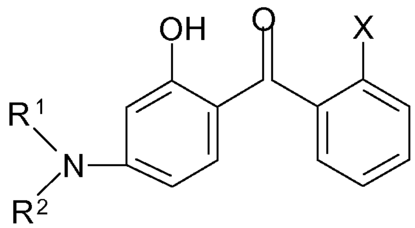

(Ia)

dans laquelle :

$R^1$ et $R^2$ , identiques ou différents, désignent un atome d'hydrogène, un radical alkyle en $C_1$-$C_{12}$ ou forment avec l'atome d'azote avec lequel ils sont liés un cycle à 5 ou 6 chaînons ;
X désigne $COOR^5$ ou $CONR^6R^7$;
$R^5$ désigne un atome d'hydrogène, un radical alkyle en $C_1$-$C_{12}$, un radical cycloalkyle en $C_3$-$C_6$.
$R^6$ et $R^7$ , identiques ou différents, désignent un atome d'hydrogène, un radical alkyle en $C_1$-$C_{12}$, un radical cycloalkyle en $C_5$-$C_6$.

Les composés de formule (Ia) plus particulièrement préférés sont ceux pour lesquels :

$R^1$ et $R^2$, identiques ou différents, désignent un radical alkyle en $C_1$-$C_4$ et plus particulèrement éthyle ;
$R^5$ désigne un radical alkyle en $C_3$-$C_8$,
$R^6$ et $R^7$, identiques ou différents, désignent un radical alkyle en $C_1$-$C_8$,

Une autre famille de composés de formule (I) préférentiels comprend ceux choisis parmi ceux de formule (Ib) suivante :

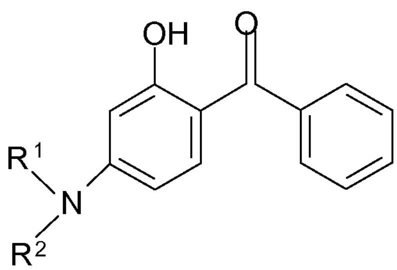

(Ib)

dans laquelle :
$R^1$ et $R^2$, identiques ou différents, désignent un radical alkyle en $C_1$-$C_{12}$ ou forment avec l'atome d'azote avec lequel ils sont liés un cycle à 5 ou 6 chaînons.

Parmi les composés de formule (Ib), on peut citer plus particulièrement :

- le (4-diéthylamino-2-hydroxyphényl)-phénylcétone.
- le (4-pyrrolidino-2-hydroxyphényl)-phénylcétone.

Une famille de composés de formule (I) plus particulièrement préférés comprend ceux choisis parmi ceux de formule (Ic) suivante :

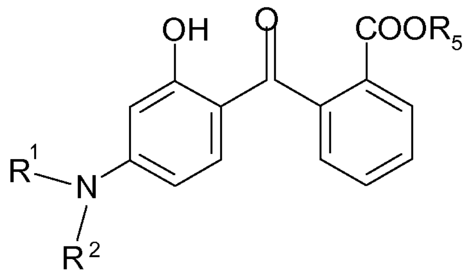

(Ic)

dans laquelle :

$R^1$ et $R^2$, identiques ou différents, désignent un atome d'hydrogène, un radical alkyle en $C_1$-$C_8$ ou forment avec l'atome d'azote avec lequel ils sont liés un cycle à 5 ou 6 chaînons ;
$R^5$ désigne un atome d'hydrogène, un radical alkyle en $C_1$-$C_{12}$, un radical cycloalkyle en $C_3$-$C_6$.

Parmi les composés de formule (Ic), on peut citer :

- le 2-(4-pyrrolidino-2-hydroxybenzoyl)-benzoate
- le 2-(4-diéthylamino-2-hydroxybenzoyl)-benzoate de méthyle
- le 2-(4-diéthylamino-2-hydroxybenzoyl)-benzoate de 2-éthylhexyle
- le 2-(4-diéthylamino-2-hydroxybenzoyl)-benzoate de cyclohexyle
- le 2-(4-diéthylamino-2-hydroxybenzoyl)-benzoate de n-hexyle
- le 2-(4-dibutylamino-2-hydroxybenzoyl)-benzoate
- le 2-(4-dibutylamino-2-hydroxybenzoyl)-benzoate de méthyle
- le 2-(4-dibutylamino-2-hydroxybenzoyl)-benzoate d'isobutyle.

Un composé de formule (I) tout particulièrement préféré est le 2-(4-diéthylamino-2-hydroxybenzoyl)-benzoate de n-hexyle.

Les composés de formule (I) tels que définis ci-dessus sont connus en eux-mêmes et leurs structures et leurs synthèses sont décrites dans les demandes de brevet EP-A-1046391 et DE100 12 408 (faisant partie intégrante du contenu de la description).

Les dérivés de 2-hydroxybenzophénone aminosubstitués conformes à l'invention sont présents de préférence dans la composition de l'invention dans des proportions allant de préférence de 0,1 à 20% en poids et plus préférentiellement de 0,1 à 15% en poids et plus particulièrement de 0,5 à 10% en poids par rapport au poids total de la composition.

Les compositions conformes à l'invention peuvent comporter en plus d'autres filtres UV organiques complémentaires actifs dans l'UVA et/ou l'UVB (absorbeurs), hydrosolubles ou liposolubles.

Les filtres UV organiques complémentaires sont notamment choisis parmi les anthranilates ; les dérivés cinnamiques ; les dérivés salicyliques, les dérivés du camphre autres que le composé (II) ; les dérivés de triazine tels que ceux décrits dans les demandes de brevet US 4367390, EP863145, EP517104, EP570838, EP796851, EP775698, EP878469 et EP933376 ; les dérivés de la benzophénone autres que ceux de formule (I) ; les dérivés de $\beta$ , $\beta$'-diphénylacrylate ; les dérivés de benzotriazole ; les dérivés de benzalmalonate ; les dérivés de benzimidazole ; les imadazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans s les demandes US5,237,071, US5,166,355, GB2303549, DE 197 26 184 et EP893119 ; les polymères filtres et silicones filtres tels que ceux décrits notamment dans la demande WO-93/04665 ; les dimères dérivés d'$\alpha$-alkylstyrène tels que ceux décrits dans la demande de brevet DE19855649; les 4,4-diarylbutadiènes tels que décrits dans les demandes EP0967200, DE19746654, DE19755649, EP-A-1008586.

Comme exemples de filtres organiques complémentaires actifs dans l'UV-A et/ou l'UV-B, on peut citer désignés ci-dessus sous leur nom INCI :

Dérivés de l'acide para-aminobenzoique :

- PABA,
- Ethyl PABA,
- Ethyl Dihydroxypropyl PABA,
- Ethylhexyl Diméthyl PABA vendu notamment sous le nom « ESCALOL 507 » par ISP,
- Glyceryl PABA,
- PEG-25 PABA vendu sous le nom « UVINUL P25 » par BASF,

Dérivés salicyliques :

- Homosalate vendu sous le nom « EUSOLEX HMS » par RONA/EM INDUSTRIES,
- Ethylhexyl Salicylate vendu sous le nom « NEO HELIOPAN OS » par HAARMANN et REIMER,
- Dipropyleneglycol Salicylate vendu sous le nom « DIPSAL » par SCHER,
- TEA Salicylate, vendu sous le nom « NEO HELIOPAN TS » par HAARMANN et REIMER,

Dérivés du dibenzovlméthane :

- Butyl Methoxydibenzoylmethane vendu notamment sous le nom commercial « PARSOL 1789 » par HOFFMANN LA ROCHE,
- Isopropyl Dibenzoylmethane,

Dérivés cinnamiques :

- Ethylhexyl Methoxycinnamate vendu notamment sous le nom commercial « PARSOL MCX » par HOFFMANN LA ROCHE,
- Isopropyl Methoxy cinnamate,
- Isoamyl Methoxy cinnamate vendu sous le nom commercial « NEO HELIOPAN E 1000 » par HAARMANN et REIMER,
- Cinoxate,
- DEA Methoxycinnamate,

    - - Diisopropyl Methylcinnamate,

- Glyceryl Ethylhexanoate Dimethoxycinnamate

Dérivés de β,β'-diphénylacrylate :

- Octocrylene vendu notamment sous le nom commercial « UVINUL N539 » par BASF,
- Etocrylene, vendu notamment sous le nom commercial « UVINUL N35 » par BASF,

Dérivés de la benzophénone :

- Benzophenone-1 vendu sous le nom commercial « UVINUL 400 » par BASF,
- Benzophenone-2 vendu sous le nom commercial « UVINUL D50 » par BASF
- Benzophenone-3 ou Oxybenzone, vendu sous le nom commercial « UVINUL M40 » par BASF,

- Benzophenone-4 vendu sous le nom commercial « UVINUL MS40 » par BASF,
- Benzophenone-5
- Benzophenone-6 vendu sous le nom commercial « HELISORB 11 » par NORQUAY
- Benzophenone-8 vendu sous le nom commercial « SPECTRA-SORB UV-24 » PAR AMERICAN CYANAMID
- Benzophenone-9 vendu sous le nom commercial« UVINUL DS-49» par BASF,
- Benzophenone-12

Dérivé du benzylidène camphre :

- 3-Benzylidene camphor fabriqué sous le nom « MEXORYL SD» par CHIMEX,
- 4-Methylbenzylidene camphor vendu sous le nom « EUSOLEX 6300 » par MERCK,
- Benzylidene Camphor Sulfonic Acid fabriqué sous le nom « MEXORYL SL» par CHIMEX,
- Camphor Benzalkonium Methosulfate fabriqué sous le nom « MEXORYL SO » par CHIMEX,
- Polyacrylamidomethyl Benzylidene Camphor fabriqué sous le nom « MEXORYL SW » par CHIMEX,

Dérivés du phenyl benzimidazole :

- Phenylbenzimidazole Sulfonic Acid vendu notamment sous le nom commercial « EUSOLEX 232 » par MERCK,
- Disodium Phenyl Dibenzimidazole Tetra-sulfonate vendu sous le nom commercial commercial « NEO HELIOPAN AP » par HAARMANN et REIMER,

Dérivés de la triazine :

- Anisotriazine vendu sous le nom commercial «TINOSORB S » par CIBA SPECIALTY CHEMICALS
- Ethylhexyl triazone vendu notamment sous le nom commercial «UVINUL T150 » par BASF,
- Diethylhexyl Butamido Triazone vendu sous le nom commercial « UVASORB HEB » par SIGMA 3V,
- la 2,4,6-tris-(4'-amino benzalmalonate de diisobutyle)-s- triazine.

Dérivés du phenyl benzotriazole :

- Drometrizole Trisiloxane vendu sous le nom « SILATRIZOLE » par RHODIA CHIMIE,
- Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

Dérivés anthraniliques :

- Menthyl anthranilate vendu sous le nom commercial commercial « NEO HELIOPAN MA » par HAARMANN et REIMER,

Dérivés d'imidazolines :

- Ethylhexyl Dimethoxybenzylidene Dioxoimidazoline Propionate,

Dérivés de benzalmalonate :

- Polyorganosiloxane à fonction benzalmalonate vendu sous la dénomination commerciale « PARSOL SLX » par HOFFMANN LA ROCHE et leurs mélanges.

Dérivés de 4,4-diarylbutadiene

1,1-dicarboxy (2,2'-diméthyl-propyl)-4,4-diphénylbutadiène.

Les filtres UV organiques solubles plus particulièrement préférés sont choisis parmi les composés suivants :

- Ethylhexyl Salicylate,
- Octocrylene,
- Ethylhexyl Methoxycinnamate
- Butyl Methoxydibenzoylmethane,
- Phenylbenzimidazole Sulfonic Acid,
- Benzophenone-3,
- Benzophenone-4,
- Benzophenone-5,
- 4-Methylbenzylidene camphor,
- Disodium Phenyl Dibenzimidazole Tetra-sulfonate,
- Anisotriazine,
- Ethylhexyl triazone,
- Diethylhexyl Butamido Triazone,
- la 2,4,6-tris-(4'-amino benzalmalonate de diisobutyle)-s- triazine.
- Drometrizole Trisiloxane,
- Methylène bis-Benzotriazolyl Tetramethylbutylphénol
- 1,1-dicarboxy (2,2'-diméthyl-propyl)-4,4-diphénylbutadiène

et leurs mélanges.

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les compositions selon l'invention peuvent également contenir des agents de bronzage et/ou de brunissage artificiels de la peau (agents autobronzants), tels que par exemple de la dihydroxyacétone (DHA).

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants, les agents anti radicaux libres, les opacifiants, les stabilisants, les émollients, les silicones, les $\alpha$-hydroxyacides, les agents anti-mousse, les agents hydratants, les vitamines, les agents répulsifs contre les insectes, les parfums, les conservateurs, les tensioactifs, les anti-inflammatoires, les antagonistes de substance P, les charges, les polymères, les propulseurs, les agents alcalinisants ou acidifiants, les colorants ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C. Ils comprennent également les acides gras, les alcools gras et les esters d'acides gras, linéaires ou cycliques tels que les dérivés d'acide benzoïque, trimellitique et hydroxy-benzoïque.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en $C_{12}$-$C_{15}$ vendu sous la dénomination commerciale « Finsolv TN » par la société Finetex, le palmitate d'octyle, le lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs.

Bien entendu, l'homme de l'art veillera à choisir ce ou ces éventuels composés complémentaires et/ou leurs quantités de manière telle que les propriétés avantageuses, en particulier l'effet de synergie, attachées intrinsèquement aux compositions conformes à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Ces compositions peuvent se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de poudre, de stick, de mousse aérosol ou de spray.

Lorsque la composition cosmétique selon l'invention est utilisée pour la protection des cheveux contre les rayons UV, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

## Revendications

1. Composition cosmétique ou dermatologique à usage topique, en particulier pour la photoprotection de la peau et/ou des cheveux, **caractérisée par le fait qu'**elle comprend, dans un support cosmétiquement acceptable :

   (a) à titre de premier filtre, au moins un filtre UV organique, insoluble de taille de particule allant de 10 nm à 5 μm et choisi parmi les filtres UV organiques du type oxalanilide ; du type triazine ; du type amide vinylique ; du type cinnamamide ; du type comportant un ou plusieurs groupements benzazole et/ou benzofuranne, benzothiophène ou du type indole ; du type aryl vinylène cétone ; du type dérivé de phénylène bis-benzoxazinone du type dérivé amide, sulfonamide ou carbamate d'acrylonitrile ; les sels de métaux polyvalents de filtres UV organiques sulfoniques ou carboxyliques et

   (b) à titre de second filtre, au moins un dérivé de 2-hydroxybenzophénone amino-substitué de formule (I) suivante :

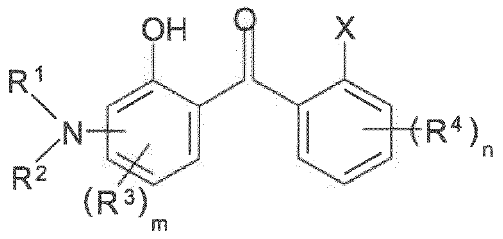

(I)

dans laquelle :

$R^1$ et $R^2$, identiques ou différents, désignent un atome d'hydrogène, un radical alkyle en $C_1$-$C_{20}$, un radical alcènyle en $C_2$-$C_{10}$, un radical cycloalkyle en $C_3$-$C_{10}$, un radical cycloalcènyle en $C_3$-$C_{10}$ ;
$R^1$ et $R^2$ peuvent également former avec l'atome d'azote avec lequel ils sont liés un cycle à 5 ou 6 chaînons ;
$R^3$ et $R^4$, identiques ou différents, désignent un radical alkyle en $C_1$-$C_{20}$, un radical alcènyle en $C_2$-$C_{10}$, un radical cycloalkyle en $C_3$-$C_{10}$, un radical cycloalcènyle en $C_3$-$C_{10}$, un radical alcoxy en $C_1$-$C_{12}$, un radical ($C_1$-$C_{20}$)alcoxycarbonyle, un radical akylamino en $C_1$-$C_{12}$, un radical dialkylamino en $C_1$-$C_{12}$, un radical aryle ou un hétéroaryle éventuellement substitué, un substituant hydrosolubilisant choisi parmi un groupe carboxylate, un groupe sulfonate ou un reste ammonium ;
X désigne un atome d'hydrogène, un groupe $COOR^5$ ou $CONR^6R^7$;
$R^5$, $R^6$ et $R^7$, identiques ou différents, désignent un atome d'hydrogène, un radical alkyle en C1-C20, un radical alcènyle en C2-C10, un radical cycloalkyle en $C_3$-$C_{10}$, un radical cycloalcènyle en C3-C10 , un groupe -(YO)o-Z ou un groupe aryle ;
Y désigne -(CH2)2-, -(CH2)3- -(CH2)4-, -CH-(CH3)-CH2- ;
Z représente $-CH_2-CH_3$, $-CH_2CH_2CH_3$, $-CH_2-CH_2-CH_2-CH_3$, $-CH(CH_3)-CH_3$ ;
m est un entier variant de 0 à3 ;
n est un entier variant de 0 à 3 ;
o est un entier variant de 1 à 2.

**2.** Composition selon la revendication 1, où les filtres UV du type oxalanilide est de formule

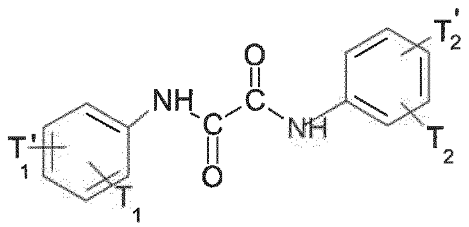

(1)

dans laquelle $T_1$ , T'1, $T_2$ et $T'_2$ désignent, identiques et différents, un atome d'hydrogène, un radical alkyle en $C_1$-$C_8$ ou un radical alcoxy en $C_1$-$C_8$.

**3.** Composition selon la revendication 1, où les filtres UV insolubles du type triazine sont choisis parmi

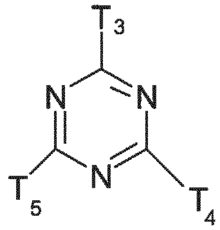

(2)

dans laquelle $T_3$, $T_4$, $T_5$, indépendamment, sont phenyle, phenoxy, pyrrolo, dans lesquels les phenyle, phenoxy, pyrrolo sont éventuellement substitués par un, deux ou trois substituants choisis parmi OH, $C_1$-$C_{18}$alkyle ou alkoxy, $C_1$-$C_{18}$carboxyalkyle, $C_5$-$C_8$cycloalkyle, un groupe méthylbenzylidènecamphre, un groupe $-(CH=CH)_n(CO)-OT_6$ , avec $T_6$ désignant $C_1$-$C_{18}$alkyle ou cinnamyle, et n vaut 0 ou 1.

**4.** Composition selon la revendication 1, où les filtres organiques du type amide vinylique, répondent à la formule suivante :

$$T_{12}\text{-}(Y)r\text{-}C(=O)\text{-}C(T_{13})=C(T_{14})\text{-}N(T_{15})(T_{16}) \qquad (5)$$

dans laquelle $T_{12}$ est un radical alkyle en $C_1$-$C_{18}$, de préférence en $C_1$-$C_5$ ou un groupe phényle éventuellement substitué par un, deux ou trois radicaux choisis parmi OH, alkyle en $C_1$-$C_{18}$, alcoxy en $C_1$-$C_8$, ou un groupe -C(=O)-$OT_{17}$ où $T_{17}$ est un alkyle en $C_1$-$C_{18}$ ; $T_{13}$, $T_{14}$, $T_{15}$ et $T_{16}$ identiques ou différents désignent un radical alkyle en $C_1$-$C_{18}$, de préférence en $C_1$-$C_5$ ou un atome d'hydrogène ; Y est N ou O et r vaut 0 ou 1.

**5.** Composition selon la revendication 1, où les filtres organiques du type cinnamamide répond à la formule suivante :

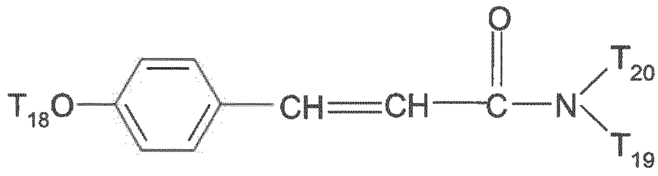

(6)

dans laquelle $OT_{18}$ est un radical hydroxy ou alcoxy en $C_1$-$C_4$, de préférence méthoxy ou éthoxy ; $T_{19}$ est hydrogène, alkyle en $C_1$-$C_4$, de préférence méthyle ou éthyle ; $T_{20}$ est un groupe -(CONH)s-phényle ou s vaut 0 ou 1 et le groupe phényle peut être substitué par un, deux ou trois groupes choisis parmi OH, alkyle en $C_1$-$C_{18}$, alcoxy en $C_1$-$C_8$, ou un groupe -C(=O)-$OT_{21}$ où $T_{21}$ est un alkyle en $C_1$-$C_{18}$.

**6.** Composition selon la revendication 1, où les filtres UV insolubles du type benzazole sont choisis parmi ceux répondant à l'une des formules (7), (8) et (9) suivantes :

(7)

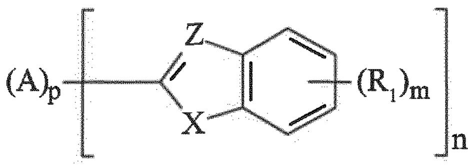

(8)

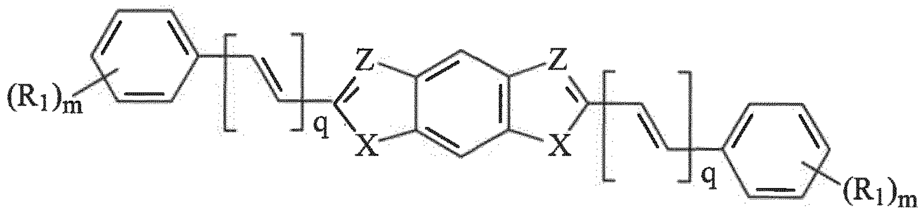

(9)

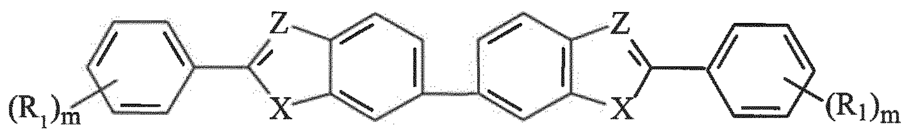

dans lesquelles

chacun des symboles X représente indépendamment un atome d'oxygène ou de soufre ou un groupe $NR_2$,
chacun des symboles Z représente indépendamment un atome d'azote ou un groupe CH,
chacun des symboles $R_1$ représente indépendamment un groupe OH, un atome d'halogène, un groupe alkyle en $C_{1-8}$, linéaire ou ramifié, contenant éventuellement un atome de silicium, ou un groupe alcoxy en $C_{1-8}$, linéaire ou ramifié,
chacun des nombres m vaut indépendamment 0, 1 ou 2,
n représente un nombre entier compris entre 1 et 4 inclus,
p est égal à 0 ou 1,

chacun des nombres q est égal indépendamment à 0 ou 1,
chacun des symboles $R_2$ représente indépendamment un atome d'hydrogène, un groupe benzyle ou alkyle en $C_{1-8}$, linéaire ou ramifié, contenant éventuellement un atome de silicium,
A représente un radical de valence n choisi parmi ceux de formules

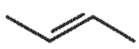
(a)
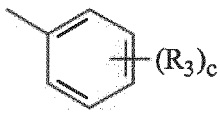
(b)
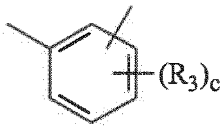
(c)
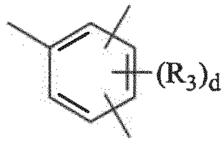
(d)
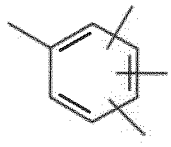
(e)

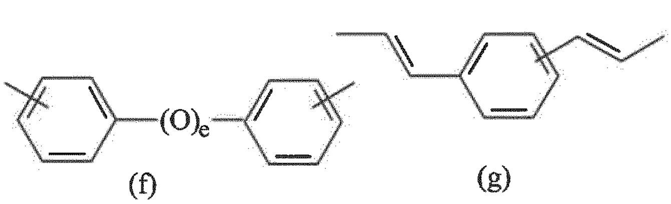
(f) (g)
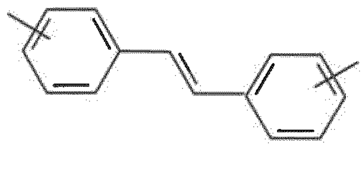
(h)

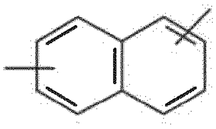
(i)
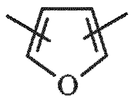
(j)
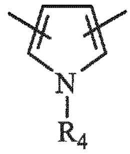
(k)
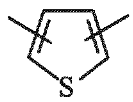
(l)

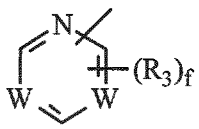
(m)
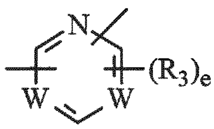
(n)
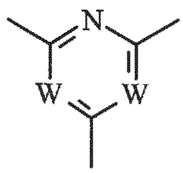
(o)

dans lesquelles :

W désigne N ou CH ; chacun des symboles $R_3$ représente indépendamment un atome d'halogène ou un groupe alkyle ou alcoxy en $C_{1-4}$, linéaire ou ramifié, ou hydroxy,
$R_4$ représente un atome d'hydrogène ou un groupe alkyle en $C_{1-4}$, linéaire ou ramifié, c = 0 - 4, d = 0 - 3, e = 0 ou 1, et f = 0-2.

**7.** Composition selon la revendication 1, où les filtres organiques insolubles du type aryl vinylène cétone répondent à l'une des formules (10) et (11) suivantes :

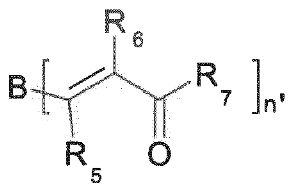
(10)

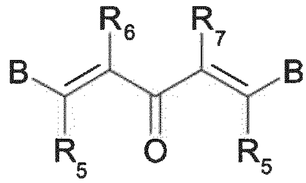

(11)

dans lesquelles :

n' = 1 ou 2,

B, dans la formule (10) lorsque n'=1 ou dans la formule (11), est un radical aryle choisi parmi les formules (a') à (d') suivantes, ou dans la formule (10) lorsque n'=2, est un radical choisi parmi les formules (e') à (h') suivantes :

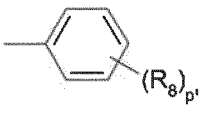
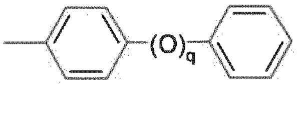
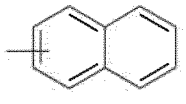
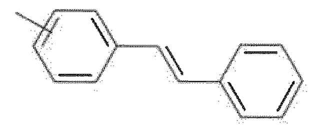

(a') (b') (c') (d')

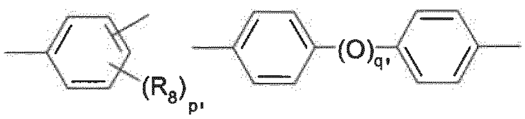
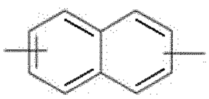
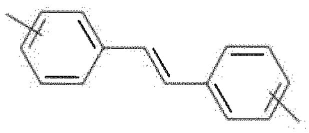

(e') (f') (g') (h')

dans lesquelles :

chacun des symboles $R_8$ représente indépendamment un groupe OH, un atome d'halogène, un groupe alkyle en $C_{1-6}$, linéaire ou ramifié et contenant éventuellement un atome de silicium, un groupe alcoxy en $C_{1-6}$, linéaire ou ramifié et contenant éventuellement un atome de silicium, un groupe alcoxycarbonyle en $C_{1-5}$, linéaire ou ramifié, ou un groupe alkylsulfonamide en $C_{1-6}$, linéaire ou ramifié et contenant éventuellement un atome de silicium ou une fonction aminoacide,

p' représente un nombre entier compris entre 0 et 4 inclus,

q' représente 0 ou 1,

$R_5$ représente l'hydrogène ou un groupe OH,

$R_6$ représente l'hydrogène, un groupe alkyle en $C_{1-6}$, linéaire ou ramifié et contenant éventuellement un atome de silicium, un groupe cyano, un groupe alkylsulfonyle en $C_{1-6}$, un groupe phénylsulfonyle,

$R_7$ représente un groupe alkyle en $C_{1-6}$, linéaire ou ramifié et contenant éventuellement un atome de silicium ou un groupe phényle pouvant former un bicycle et éventuellement substitué par un ou deux radicaux $R_4$,

ou $R_6$ et $R_7$ forment ensemble un reste hydrocarboné en $C_{2-10}$ monocyclique, bicyclique ou tricylique, éventuellement interrompu par un ou des atomes d'azote, de soufre et d'oxygène et pouvant contenir un autre carbonyle, et éventuellement substitué par un groupe alkylsulfonamide en $C_1$-$C_8$, linéaire ou ramifié, et contenant éventuellement un atome de silicium ou une fonction aminoacide ; à condition que lorsque n'=1, $R_6$ et $R_7$ ne forment pas un noyau camphre.

**8.** Composition selon la revendication 1 où les filtres organiques insolubles du type phénylène bis-benzoxazinone répondent à la formule (12) suivante :

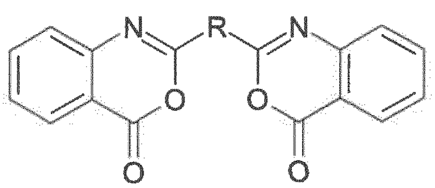

(12)

avec R représentant un reste aromatique divalent choisi parmi les formules (e") à (h") suivantes :

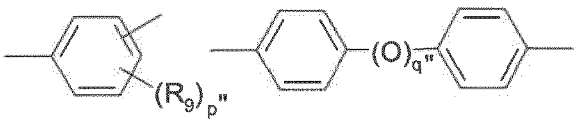

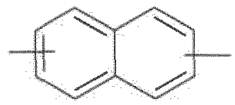

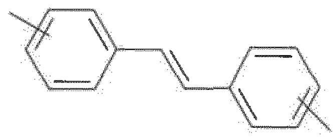

(e") (f") (g") (h")

dans lesquelles :

chacun des symboles $R_9$ représente indépendamment un groupe OH, un atome d'halogène, un groupe alkyle en $C_{1-6}$, linéaire ou ramifié et contenant éventuellement un atome de silicium, un groupe alcoxy en $C_{1-6}$, linéaire ou ramifié et contenant éventuellement un atome de silicium, un groupe alcoxycarbonyle en $C_{1-5}$, linéaire ou ramifié, ou un groupe alkylsulfonamide en $C_{1-6}$, linéaire ou ramifié et contenant éventuellement un atome de silicium ou une fonction aminoacide,
p" représente un nombre entier compris entre 0 et 4 inclus,
q" représente 0 ou 1,

**9.** Composition selon la revendication 1, où les filtres organiques insolubles du type dérivé amide, sulfonamide ou carbamate d'acrylonitrile sont choisis parmi ceux répondant à la formule (13) suivante :

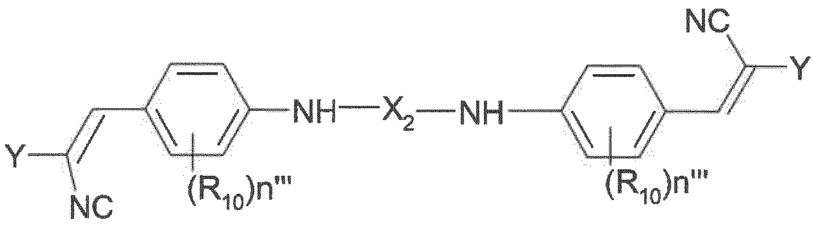

(13)

dans laquelle :

$R_{10}$ représente un groupe alkyle en $C_{1-8}$, linéaire ou ramifié,
n''' vaut 0, 1 ou 2,
$X_2$ représente un radical divalent de formule $-(C=O)-R_{11}-(C=O)-$ , $-SO_2-R_{11}-SO_2-$ ou $-(C=O)-O-R_{11}-O-(C=O)-$,
Y représente un radical $-(C=O)-R_{12}$ ou $-SO_2R_{13}$,
$R_{11}$ représente une simple liaison ou un radical divalent alkylène en $C_1$-$C_{30}$ ou alcénylène en $C_3$-$C_{30}$, linéaire ou ramifié, pouvant porter un ou plusieurs substituants hydroxyle et pouvant contenir, dans la chaîne carbonée, un ou plusieurs hétéroatomes choisis parmi les atomes d'oxygène, d'azote et de silicium,.
$R_{12}$ représente un radical $-OR_{14}$ ou $-NHR_{14}$,
$R_{13}$ représente un radical alkyle en $C_1$-$C_{30}$, linéaire ou ramifié, ou un noyau phényle non substitué ou substitué par des radicaux alkyle ou alcoxy en $C_1$-$C_4$,
$R_{14}$ représente un radical alkyle en $C_1$-$C_{30}$ ou alcényle en $C_3$-$C_{30}$, linéaire ou ramifié, pouvant porter un ou plusieurs substituants hydroxyle et pouvant contenir,
dans la chaîne carbonée, un ou plusieurs hétéroatomes choisis parmi les atomes d'oxygène, d'azote et de silicium ; ainsi que leurs isomères.

**10.** Composition selon la revendication 1, où les filtres UV insolubles sont choisis parmi les sels de métaux polyvalents de dérivés sulfonés de benzylidène camphre ; les sels de métaux polyvalents de dérivés sulfonés de benzimidazole ; les sels de métaux polyvalents de dérivés d'acide cinnamique.

**11.** Composition selon l'une quelcconque des revendications 1 à 10, où le ou les composés de formule (I) sont choisis parmi ceux de formule (Ic) suivante :

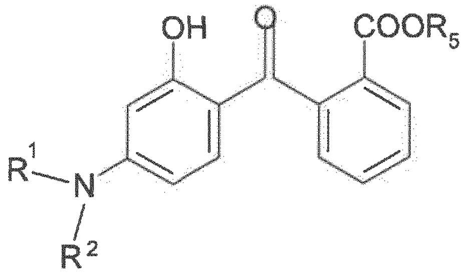

(Ic)

dans laquelle :

$R^1$ et $R^2$, identiques ou différents, désignent un atome d'hydrogène, un radical alkyle en $C_1$-$C_8$ ou forment avec l'atome d'azote avec lequel ils sont liés un cycle à 5 ou 6 chaînons ;
$R^5$ désigne un atome d'hydrogène, un radical alkyle en $C_1$-$C_{12}$, un radical cycloalkyle en $C_3$-$C_6$.

**12.** Composition selon la revendication 11 où le composé de formule (Ic) est le 2-(4-diéthylamino-2-hydroxybenzoyl)-benzoate de n-hexyle

**13.** Composition selon l'une quelconque des revendications 1 à 12, **caractérisée par le fait qu'**elle comprend en outre au moins un agent de bronzage et/ou de brunissage artificiel de la peau.

**14.** Composition selon l'une quelconque des revendications 1 à 13, **caractérisée par le fait qu'**elle comprend en outre au moins un filtre UV organique soluble actif dans l'UV-A et/ou l'UV-B.

**15.** Composition selon l'une quelconque des revendications 1 à 14, **caractérisée par le fait qu'**elle comprend en outre, des pigments d'oxydes métalliques, enrobés ou non.

**16.** Utilisation d'un dérivé de 2-hydroxybenzophénone aminosubstitué de formule (I) tel que défini dans les revendications précédentes dans une composition cosmétique destinée à la protection de la peau et/ou des cheveux contre le rayonnement ultraviolet, en particulier le rayonnement solaire comprenant au moins un filtre organique insoluble de taille de particule allant de 10 nm à 5 $\mu$m tel que défini dans les revendications précédentes, dans le but de produire un effet synergique au niveau des facteurs de protection solaire UV-$A_{PPD}$ conférés.

## Patentansprüche

**1.** Kosmetische oder dermatologische Zusammensetzung zur topischen Anwendung, insbesondere für den Sonnenschutz der Haut und/oder der Haare, **dadurch gekennzeichnet, dass** diese, in einem kosmetisch annehmbaren Träger, umfasst:

(a) als ersten Filter, mindestens einen organischen, unlöslichen UV-Filter mit einer Teilchengröße von 10 nm bis 5 $\mu$m und ausgewählt aus organischen UV-Filtern vom Typ Oxalanilid; vom Typ Triazin; vom Typ Vinylamid; vom Typ Cinnamamid; vom Typ umfassend eine oder mehrere Benzazol- und/oder Benzofuran-, Benzothiophen-Gruppen, oder vom Typ Indol; vom Typ Arylvinylenketon; vom Typ Phenylen-bis-benzoxazinon-Derivat vom Typ Amid-, Sulfonamid- oder Carbamat-Derivat von Acrylonitril; polyvalenten Metallsalzen von organischen Sulfon- oder Carboxy-UV-Filtern, und
(b) als zweiten Filter, mindestens ein substituiertes 2-Hydroxybenzophenonamino-Derivat mit der folgenden Formel (I):

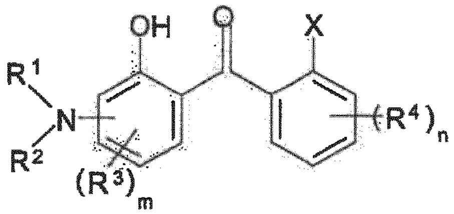

(I)

wobei:

$R^1$ und $R^2$, identisch oder verschieden, ein Wasserstoffatom, einen $C_1$-$C_{20}$-Alkylrest, einen $C_2$-$C_{10}$-Alkenylrest, einen $C_3$-$C_{10}$-Cycloalkylrest, einen $C_3$-$C_{10}$-Cycloalkenylrest darstellen;
$R^1$ und $R^2$ auch mit dem Stickstoffatom, an das sie gebunden sind, einen Ring mit 5 oder 6 Gliedern bilden können;
$R^3$ und $R^4$, identisch oder verschieden, einen $C_1$-$C_{20}$-Alkylrest, einen $C_2$-$C_{10}$-Alkenylrest, einen $C_3$-$C_{10}$-Cycloalkylrest, einen $C_3$-$C_{10}$-Cycloalkenylrest, einen $C_1$-$C_{12}$-Alkoxyrest, einen $C_1$-$C_{20}$-Alkoxycarbonylrest, einen $C_1$-$C_{12}$-Alkylaminorest, einen $C_1$-$C_{12}$-Dialkylaminorest, einen Arylrest oder ein Heteroaryl, gegebenenfalls substituiert, einen wasserlöslich machenden Substituenten, ausgewählt aus einer Carboxylatgruppe, einer Sulfonatgruppe oder einem Ammoniumrest, darstellen;
X ein Wasserstoffatom, eine Gruppe $COOR^5$ oder $CONR^6R^7$ darstellt;
$R^5$, $R^6$ und $R^7$, identisch oder verschieden, ein Wasserstoffatom, einen $C_1$-$C_{20}$-Alkylrest, einen $C_2$-$C_{10}$-Alkenylrest, einen $C_3$-$C_{10}$-Cycloalkylrest, einen $C_3$-$C_{10}$-Cycloalkenylrest, eine Gruppe -(YO)o-Z oder eine Arylgruppe darstellen;
Y - $(CH_2)_2$-, - $(CH_2)_3$- - $(CH_2)_4$-, -CH- $(CH_3)$ -$CH_2$- darstellt;
Z -$CH_2$ -$CH_3$ -, -$CH_2CH_2CH_3$ -, -$CH_2$-$CH_2$-$CH_2$-$CH_3$, - CH $(CH_3)$ - $CH_3$ darstellt;
m eine ganze Zahl variierend von 0 bis 3 ist;
n eine ganze Zahl variierend von 0 bis 3 ist;
o eine ganze Zahl variierend von 1 bis 2 ist.

**2.** Zusammensetzung nach Anspruch 1,
wobei die UV-Filter vom Typ Oxalanilid die Formel aufweisen:

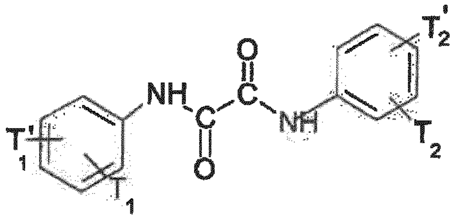

**(1)**

wobei $T_1$, $T'_1$, $T_2$ und $T'_2$, identisch oder verschieden, ein Wasserstoffatom, einen $C_1$-$C_8$-Alkylrest oder einen $C_1$-$C_8$-Alkoxyrest darstellen.

**3.** Zusammensetzung nach Anspruch 1,
wobei die unlöslichen UV-Filter vom Typ Triazin ausgewählt sind aus:

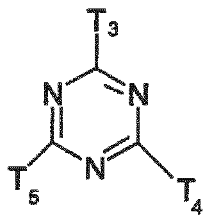

**(2)**

wobei $T_3$, $T_4$, $T_5$ unabhängig Phenyl, Phenoxy, Pyrrolo sind, wobei Phenyl, Phenoxy, Pyrrolo gegebenenfalls durch einen, zwei oder drei Substituenten substituiert sind, ausgewählt aus OH, $C_1$-$C_{18}$-Alkyl oder -Alkoxy, $C_1$-$C_{18}$-Carboxyalkyl, $C_5$-$C_8$-Cycloalkyl, einer Methylbenzylidencamphergruppe, einer Gruppe - $(CH=CH)_n(CO)$ -$OT_6$, wobei $T_6$ $C_1$-$C_{18}$-Alkyl oder -Cinnamyl darstellt, und n 0 oder 1 ist.

**4.** Zusammensetzung nach Anspruch 1, wobei die organischen Filter vom Typ Vinylamid die folgende Formel aufweisen:

**$T_{12}$-(Y)r-C(=O)-C($T_{13}$)=C($T_{14}$)-N($T_{15}$)($T_{16}$)** **(5)**

wobei $T_{12}$ ein $C_1$-$C_{18}$-, vorzugsweise $C_1$-$C_5$-Alkylrest, oder eine Phenylgruppe ist, gegebenenfalls substituiert durch

einen, zwei oder drei Reste, ausgewählt aus OH, $C_1$-$C_{18}$-Alkyl, $C_1$-$C_8$-Alkoxy, oder eine Gruppe -C(=O)-$OT_{17}$, wobei $T_{17}$ ein $C_1$-$C_{18}$-Alkyl ist; $T_{13}$, $T_{14}$, $T_{15}$ und $T_{16}$, identisch oder verschieden, einen $C_1$-$C_{18}$-, vorzugsweise $C_1$-$C_5$-Alkylrest, oder ein Wasserstoffatom darstellen; Y N oder O ist; und r 0 oder 1 ist.

**5.** Zusammensetzung nach Anspruch 1,
wobei die organischen Filter vom Typ Cinnamamid die folgende Formel aufweisen:

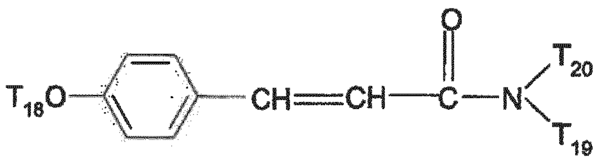

(6)

wobei $OT_{18}$ ein $C_1$-$C_4$-Hydroxy- oder -Alkoxyrest ist, vorzugsweise Methoxy oder Ethoxy; $T_{19}$ Wasserstoff, $C_1$-$C_4$-Alkyl ist, vorzugsweise Methyl oder Ethyl; $T_{20}$ eine Gruppe -(CONH)s-Phenyl ist, wobei s 0 oder 1 ist, und die Phenylgruppe substituiert sein kann durch eine, zwei oder drei Gruppen, ausgewählt aus OH, $C_1$-$C_{18}$-Alkyl, $C_1$-$C_8$-Alkoxy oder einer Gruppe -C(=O)-$OT_{21}$, wobei $T_{21}$ ein $C_1$-$C_{18}$-Alkyl ist.

**6.** Zusammensetzung nach Anspruch 1,
wobei die unlöslichen UV-Filter vom Typ Benzazol ausgewählt sind aus jenen mit einer der folgenden Formeln (7), (8) und (9):

(7)

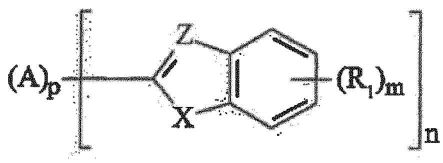

(8)

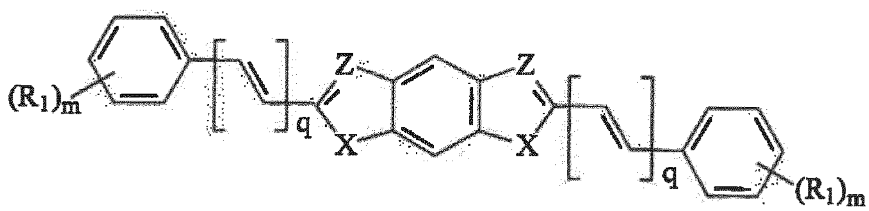

(9)

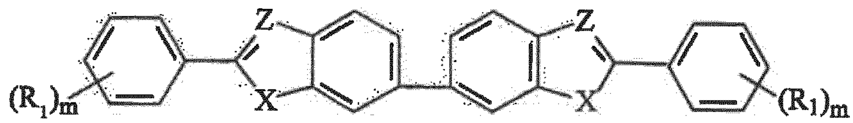

wobei:

jedes der Symbole X unabhängig ein Sauerstoff- oder Schwefelatom oder eine Gruppe $NR_2$ darstellt; jedes der Symbole Z unabhängig ein Stickstoffatom oder eine Gruppe CH darstellt;
jedes der Symbole $R_1$ unabhängig eine Gruppe OH, ein Halogenatom, eine $C_1$-$C_8$-Alkylgruppe, linear oder verzweigt, gegebenenfalls enthaltend ein Siliciumatom, oder eine $C_1$-$C_8$-Alkoxygruppe, linear oder verzweigt, darstellt;
jede der Zahlen m unabhängig 0, 1 oder 2 ist;
n eine ganze Zahl zwischen einschließlich 1 und 4 darstellt;
p gleich 0 oder 1 ist;
jede der Zahlen q unabhängig gleich 0 oder 1 ist;
jedes der Symbole $R_2$ unabhängig ein Wasserstoffatom, eine $C_1$-$C_8$-Benzyl- oder - Alkylgruppe, linear oder verzweigt, gegebenenfalls enthaltend ein Siliciumatom, darstellt;
A einen Rest mit der Wertigkeit n darstellt, ausgewählt aus jenen mit den Formeln:

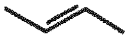
(a)

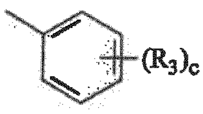
(b)

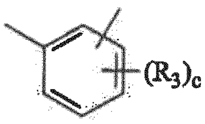
(c)

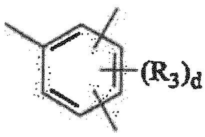
(d)

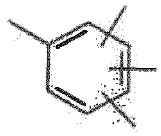
(e)

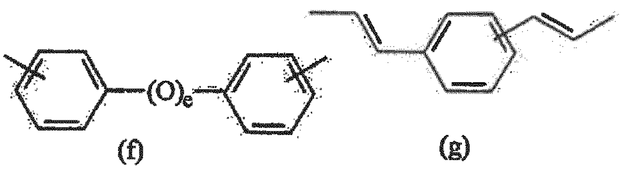
(f) (g)

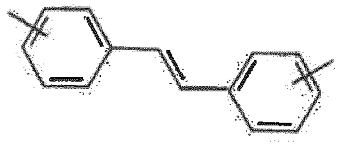
(h)

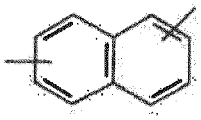
(i)

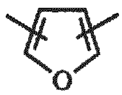
(j)

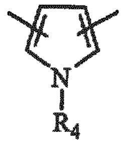
(k)

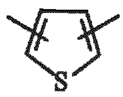
(l)

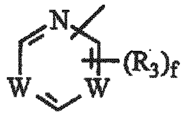
(m)

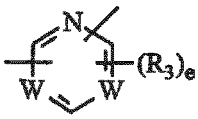
(n)

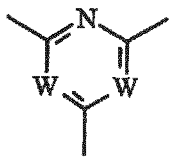
(o)

wobei:

W N oder CH darstellt; jedes der Symbole $R_3$ unabhängig ein Halogenatom oder eine $C_1$-$C_4$-Alkyl- oder -Alkoxygruppe, linear oder verzweigt, oder Hydroxy darstellt;
$R_4$ ein Wasserstoffatom oder eine $C_1$-$C_4$-Alkylgruppe, linear oder verzweigt, darstellt; c = 0 - 4; d = 0 - 3; e = 0 oder 1; und f = 0 - 2.

**7.** Zusammensetzung nach Anspruch 1,
wobei die unlöslichen organischen Filter vom Typ Arylvinylenketon eine der folgenden Formeln (10) und (11) aufweisen:

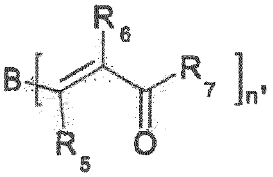
(10)

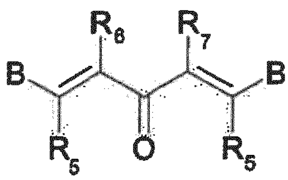
(11)

wobei:

n' = 1 oder 2;

B, in der Formel (10), wenn n' = 1, oder in der Formel (11), ein Arylrest ist, ausgewählt aus den folgenden Formeln (a') bis (d'), oder in der Formel (10), wenn n' = 2, ein Rest ist, ausgewählt aus den folgenden Formeln (e') bis (h'):

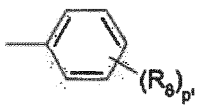

(a')

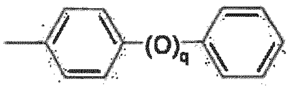

(b')

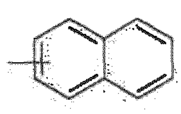

(c')

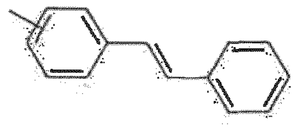

(d')

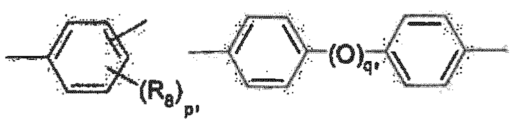

(e') (f')

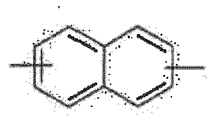

(g')

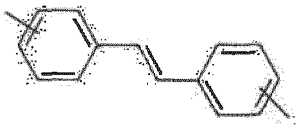

(h')

wobei:

jedes der Symbole $R_8$ unabhängig eine Gruppe OH, ein Halogenatom, eine $C_1$-$C_6$-Alkylgruppe, linear oder verzweigt, und gegebenenfalls enthaltend ein Siliciumatom, eine $C_1$-$C_6$-Alkoxygruppe, linear oder verzweigt, und gegebenenfalls enthaltend ein Siliciumatom, eine $C_1$-$C_5$-Alkoxycarbonylgruppe, linear oder verzweigt, oder eine $C_1$-$C_6$-Alkylsulfonamidgruppe, linear oder verzweigt, und gegebenenfalls enthaltend ein Siliciumatom oder eine Aminosäurefunktion, darstellt;

p' eine ganze Zahl zwischen einschließlich 0 und 4 darstellt;

q' 0 oder 1 darstellt;

$R_5$ Wasserstoff oder eine Gruppe OH darstellt;

$R_6$ Wasserstoff, eine $C_1$-$C_6$-Alkylgruppe, linear oder verzweigt, und gegebenenfalls enthaltend ein Siliciumatom, eine Cyanogruppe, eine $C_1$-$C_6$-Alkylsulfonylgruppe, eine Phenylsulfonylgruppe darstellt;

$R_7$ eine $C_1$-$C_6$-Alkylgruppe, linear oder verzweigt, und gegebenenfalls enthaltend ein Siliciumatom, oder eine Phenylgruppe, die einen Bizyklus bilden kann, und gegebenenfalls substituiert durch einen oder zwei Reste $R_4$, darstellt;

wobei $R_6$ und $R_7$ gemeinsam einen monocyclischen, bicyclischen oder tricylischen $C_2$-$C_{10}$-Kohlenwasserstoffrest bilden, gegebenenfalls unterbrochen durch ein oder mehrere Stickstoff-, Schwefel- und Sauerstoffatome, und der ein anderes Carbonyl enthalten kann, und gegebenenfalls substituiert durch eine $C_1$-$C_8$-Alkylsulfonamidgruppe, linear oder verzweigt, und gegebenenfalls enthaltend ein Siliciumatom oder eine Aminosäurefunktion; mit der Maßgabe, dass, wenn n' = 1, $R_6$ und $R_7$ keinen Campherkern bilden.

**8.** Zusammensetzung nach Anspruch 1,
wobei die unlöslichen organischen Filter vom Typ Phenylen-bis-benzooxazinon die folgende Formel (12) aufweisen:

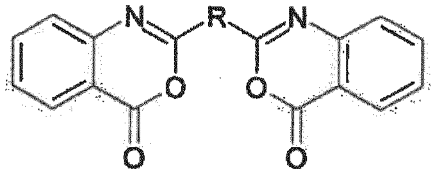

(12)

wobei R einen divalenten aromatischen Rest darstellt, ausgewählt aus den folgenden Formeln (e") bis (h"):

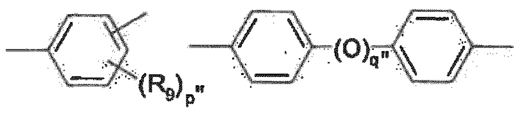

(e") (f")

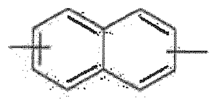

(g")

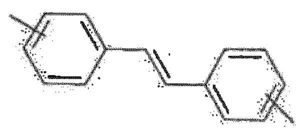

(h")

wobei:

jedes der Symbole $R_9$ unabhängig eine Gruppe OH, ein Halogenatom, eine $C_1$-$C_6$-Alkylgruppe, linear oder verzweigt, und gegebenenfalls enthaltend ein Siliciumatom, eine $C_1$-$C_6$-Alkoxygruppe, linear oder verzweigt, und gegebenenfalls enthaltend ein Siliciumatom, eine $C_1$-$C_5$-Alkoxycarbonylgruppe, linear oder verzweigt, oder eine $C_1$-$C_6$-Alkylsulfonamidgruppe, linear oder verzweigt, und gegebenenfalls enthaltend ein Siliciumatom oder eine Aminosäurefunktion, darstellt;
p" eine ganze Zahl zwischen einschließlich 0 und 4 darstellt;
q" 0 oder 1 darstellt.

**9.** Zusammensetzung nach Anspruch 1,
wobei die unlöslichen organischen Filter vom Typ Amid-, Sulfonamid- oder Carbamat-Derivat von Acrylonitril ausgewählt sind aus jenen mit der folgenden Formel (13):

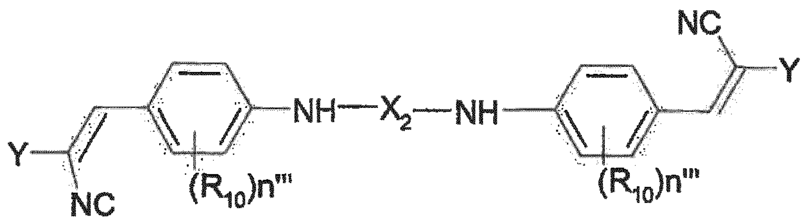

(13)

wobei:

$R_{10}$ eine $C_1$-$C_8$-Alkylgruppe, linear oder verzweigt, darstellt;
n''' 0, 1 oder 2 ist;
$X_2$ einen divalenten Rest mit der Formel -(C=O)-$R_{11}$-(C=O)-, -$SO_2$-$R_{11}$-$SO_2$- oder -(C=O) -O-$R_n$-O (C=O) - darstellt;
Y einen Rest - (C=O) -$R_{12}$ oder -$SO_2R_{13}$ darstellt;
$R_{11}$ eine Einfachbindung oder einen divalenten $C_1$-$C_{30}$-Alkylen- oder $C_3$-$C_{30}$-Alkenylrest, linear oder verzweigt, darstellt, der einen oder mehrere Hydroxyl-Substituenten tragen kann, und, in der Kohlenstoffkette, ein oder mehrere Heteroatome enthalten kann, ausgewählt aus Sauerstoff-, Stickstoff- und Siliciumatomen;
$R_{12}$ einen Rest -$OR_{14}$ oder -$NHR_{14}$ darstellt;
$R_{13}$ einen $C_1$-$C_{30}$-Alkylrest, linear oder verzweigt, oder einen Phenylkern, unsubstituiert oder substituiert durch $C_1$-$C_4$-Alkyl- oder -Alkoxyreste, darstellt;
$R_{14}$ einen $C_1$-$C_{30}$-Alkylrest oder $C_3$-$C_{30}$-Alkenylrest, linear oder verzweigt, darstellt, der einen oder mehrere Hydroxyl-Substituenten tragen kann, und, in der Kohlenstoffkette, ein oder mehrere Heteroatome enthalten kann, ausgewählt aus Sauerstoff-, Stickstoff- und Siliciumatomen; sowie ihre Isomere.

**10.** Zusammensetzung nach Anspruch 1,
wobei die unlöslichen UV-Filter ausgewählt sind aus polyvalenten Metallsalzen von Benzyliden camphersulfon-Derivaten; polyvalenten Metallsalzen von Benzimidazolsulfon-Derivaten; polyvalenten Metallsalzen von Zimtsäure-Derivaten.

**11.** Zusammensetzung nach einem der Ansprüche 1 bis 10, wobei die Verbindung oder Verbindungen mit der Formel (I) ausgewählt sind aus jenen mit der folgenden Formel (Ic):

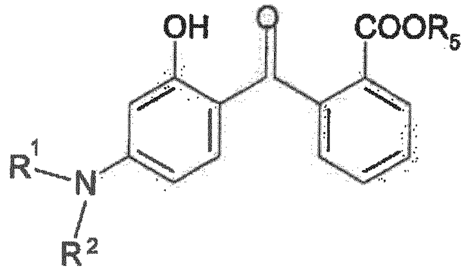

(Ic)

wobei:

$R^1$ und $R^2$, identisch oder verschieden, ein Wasserstoffatom, einen $C_1$-$C_8$-Alkylrest darstellen, oder mit dem Stickstoffatom, an das sie gebunden sind, einen Ring mit 5 oder 6 Gliedern bilden;
$R_5$ ein Wasserstoffatom, einen $C_1$-$C_{12}$-Alkylrest, einen $C_3$-$C_6$-Cycloalkylrest darstellt.

**12.** Zusammensetzung nach Anspruch 11,
wobei die Verbindung mit der Formel (Ic) N-Hexyl-2-(4-diethylamino-2-hydroxybenzoyl)-benzoat ist.

**13.** Zusammensetzung nach einem der Ansprüche 1 bis 12, **dadurch gekennzeichnet, dass** diese außerdem mindestens ein Bräunungsmittel und/oder künstliches Braunfärbungsmittel der Haut umfasst.

**14.** Zusammensetzung nach einem der Ansprüche 1 bis 13, **dadurch gekennzeichnet, dass** diese außerdem mindestens einen organischen löslichen UV-Filter umfasst, der in UV-A und/oder UV-B aktiv ist.

**15.** Zusammensetzung nach einem der Ansprüche 1 bis 14, **dadurch gekennzeichnet, dass** diese außerdem Metalloxidpigmente, beschichtet oder nicht, umfasst.

**16.** Verwendung eines Derivats von aminosubstituiertem 2-Hydroxybenzophenon mit der Formel (I), wie in den vorhergehenden Ansprüchen definiert, in einer kosmetischen Zusammensetzung, die für den Schutz der Haut und/oder der Haare gegen Ultraviolettstrahlung bestimmt ist, insbesondere gegen Sonnenstrahlung, umfassend mindestens einen unlöslichen organischen Filter mit einer Teilchengröße von 10 nm bis 5 µm, wie in den vorhergehenden Ansprüchen definiert, mit dem Ziel, einen synergistischen Effekt auf der Ebene der verliehenen Faktoren eines UV-$A_{PPD}$-Sonnenschutzes zu erzeugen.

## Claims

**1.** Cosmetic or dermatological composition for topical use, in particular for photoprotecting the skin and/or the hair, **characterized in that** it comprises, in a cosmetically acceptable support:

(a) as first screening agent, at least one insoluble organic UV-screening agent with a particle size ranging from 10 nm to 5 µm, and chosen from organic UV-screening agents of the oxalanilide type; of the triazine type of the vinyl amide type; of the cinnamamide type; of the type comprising one or more benzazole and/or benzofuran or benzothiophene groups or of the indole type; of the aryl vinylene ketone type; of the phenylene bis-benzoxazinone derivative type of the amide, sulfonamide or carbamate acrylonitrile derivative type; the polyvalent metal salts of sulfonic or carboxylic organic UV-screening agents, and
(b) as second screening agent, at least one amino-substituted 2-hydroxybenzophenone derivative of formula (I) below:

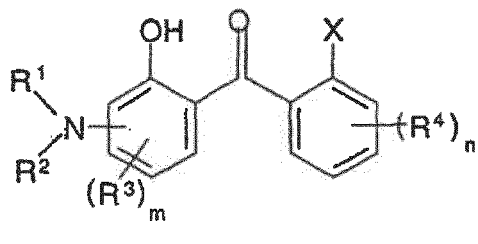

(I)

in which:

$R^1$ and $R^2$, which may be identical or different, denote a hydrogen atom, a $C_1$-$C_{20}$ alkyl radical, a $C_2$-$C_{10}$ alkenyl radical, a $C_3$-$C_{10}$ cycloalkyl radical or a $C_3$-$C_{10}$ cycloalkenyl radical;
$R^1$ and $R^2$ may also form, with the nitrogen atom to which they are attached, a 5- or 6-membered ring;
$R^3$ and $R^4$, which may be identical or different, denote a $C_1$-$C_{20}$ alkyl radical, a $C_2$-$C_{10}$ alkenyl radical, a $C_3$-$C_{10}$ cycloalkyl radical, a $C_3$-$C_{10}$ cycloalkenyl radical, a $C_1$-$C_{12}$ alkoxy radical, a ($C_1$-$C_{20}$) alkoxycarbonyl radical, a $C_1$-$C_{12}$ alkylamino radical, a $C_1$-$C_{12}$ dialkylamino radical, an optionally substituted aryl or heteroaryl radical, a hydro-solubilizing substituent chosen from a carboxylate group, a sulfonate group and ammonium residue;
X denotes a hydrogen atom or a group $COOR^5$ or $CONR^6R^7$;
$R^5$, $R^6$ and $R^7$, which may be identical or different, denote a hydrogen atom, a $C_1$-$C_{20}$ alkyl radical, a $C_2$-$C_{10}$ alkenyl radical, a $C_3$-$C_{10}$ cycloalkyl radical, a $C_3$-$C_{10}$ cycloalkenyl radical, a group -(YO)o-Z or an aryl group;
Y denotes $-(CH_2)_2-$, $-(CH_2)_3-$, $-(CH_2)_4-$ or $-CH(CH_3)-CH_2-$;
Z represents $-CH_2-CH_3$, $-CH_2CH_2CH_3$, $-CH_2-CH_2-CH_2-CH_3$ or $-CH(CH_3)CH_3$;
m is an integer ranging from 0 to 3;
n is an integer ranging from 0 to 3;
o is an integer ranging from 1 to 2.

**2.** Composition according to Claim 1, in which the UV-screening agents of the oxalanilide type are of formula:

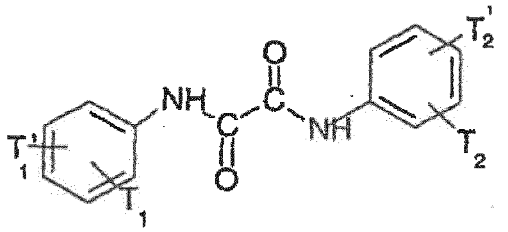

(1)

in which $T_1$, $T'_1$, $T_2$ and $T'_2$, which may be identical or different, denote a hydrogen atom, a $C_1$-$C_8$ alkyl radical or a $C_1$-$C_8$ alkoxy radical.

**3.** Composition according to Claim 1, in which the insoluble UV-screening agents of the triazine type are chosen from:

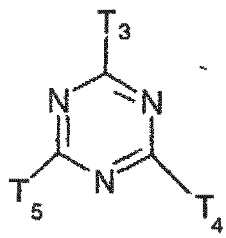

(2)

in which $T_3$, $T_4$ and $T_5$, independently, are phenyl, phenoxy or pyrrolo, in which the phenyl, phenoxy and pyrrolo are optionally substituted with one, two or three substituents chosen from OH, $C_1$-$C_{18}$alkyl or alkoxy, $C_1$-$C_{18}$carboxyalkyl, $C_5$-$C_8$cycloalkyl, a methyl-benzylidenecamphor group, a group $-(CH=CH)_n(CO)-OT_6$, with $T_6$ denoting $C_1$-$C_{18}$alkyl or cinnamyl, and n is 0 or 1.

**4.** Composition according to Claim 1, in which the organic screening agents of the vinyl amide type correspond to the following formula:

$$T_{12}-(Y)r-C(=O)-C(T_{13})=C(T_{14})-N(T_{15})(T_{16}) \qquad (5)$$

in which $T_{12}$ is a $C_1$-$C_{18}$ and preferably $C_1$-$C_5$ alkyl radical or a phenyl group optionally substituted with one, two or three radicals chosen from OH, $C_1$-$C_{18}$ alkyl, $C_1$-$C_8$ alkoxy and a group $-C(=O)-OT_{17}$ in which $T_{17}$ is a $C_1$-$C_{18}$ alkyl; $T_{13}$, $T_{14}$, $T_{15}$ and $T_{16}$, which may be identical or different, denote a $C_1$-$C_{18}$ and preferably $C_1$-$C_5$ alkyl radical or a hydrogen atom; Y is N or O and r is 0 or 1.

**5.** Composition according to Claim 1, in which the organic screening agents of the cinnamamide type correspond to the following formula:

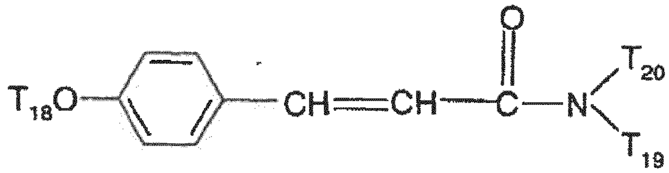

(6)

in which $OT_{18}$ is a hydroxyl or $C_1$-$C_4$ alkoxy radical, preferably methoxy or ethoxy; $T_{19}$ is hydrogen or $C_1$-$C_4$ alkyl, preferably methyl or ethyl; $T_{20}$ is a group - (CONH) s-phenyl in which s is 0 or 1 and the phenyl group may be substituted with one, two or three groups chosen from OH, $C_1$-$C_{18}$ alkyl, $C_1$-$C_8$ alkoxy and a group -C(=O)-$OT_{21}$ in which $T_{21}$ is a $C_1$-$C_{18}$ alkyl.

**6.** Composition according to Claim 1, in which the insoluble UV-screening agents of the benzazole type are chosen from those corresponding to one of the formulae (7), (8) and (9) below:

(7)

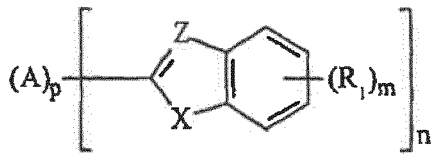

(8)

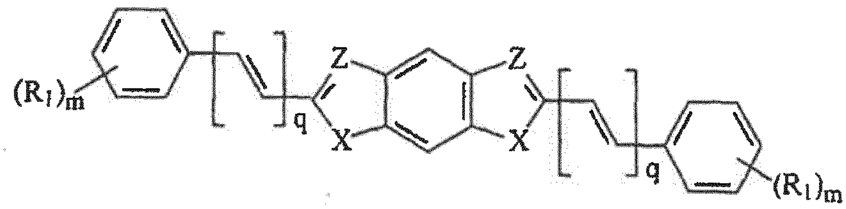

(9)

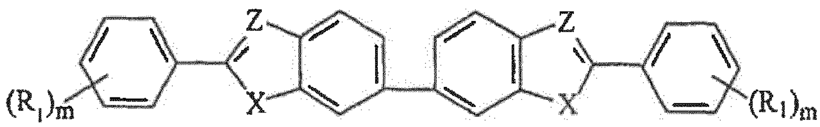

in which:

each of the symbols X independently represents an oxygen or sulphur atom or a group $NR_2$,
each of the symbols Z independently represents a nitrogen atom or a CH group,
each of the symbols $R_1$ independently represents an OH group, a halogen atom, a linear or branched $C_{1-8}$ alkyl group, optionally containing a silicon atom, or a linear or branched $C_{1-8}$ alkoxy group,
each of the numbers m is independently 0, 1 or 2,
n represents an integer between 1 and 4 inclusive,
p is equal to 0 or 1,
each of the numbers q is independently equal to 0 or 1,
each of the symbols $R_2$ independently represents a hydrogen atom or a benzyl or linear or branched $C_{1-8}$ alkyl group, optionally containing a silicon atom,
A represents a radical of valency n chosen from those of formulae:

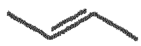
(a)

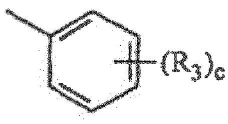
(b)

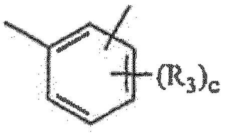
(c)

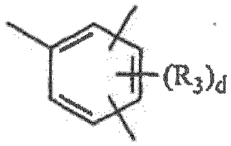
(d)

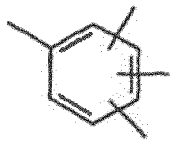
(e)

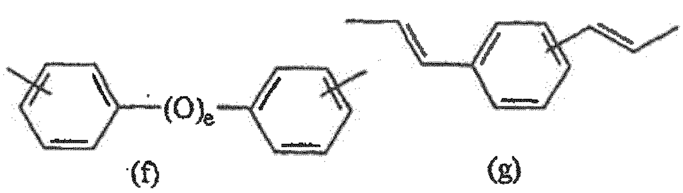
(f)

(g)

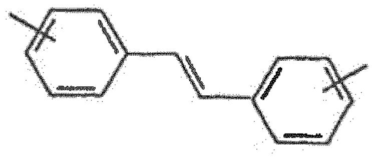
(h)

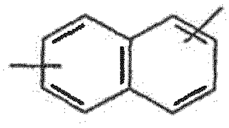
(i)

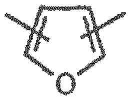
(j)

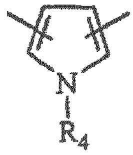
(k)

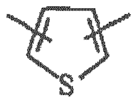
(l)

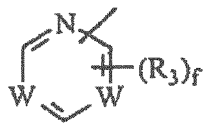
(m)

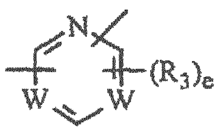
(n)

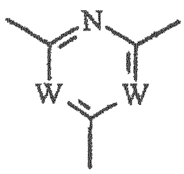
(o)

in which:

W denotes N or CH; each of the symbols $R_3$ independently represents a halogen atom or a linear or branched $C_{1-4}$ alkyl or alkoxy group, or hydroxyl,

$R_4$ represents a hydrogen atom or a linear or branched $C_{1-4}$ alkyl group, c = 0-4, d = 0-3, e = 0 or 1 and f = 0-2.

**7.** Composition according to Claim 1, in which the insoluble organic screening agents of the aryl vinylene ketone type correspond to one of the formulae (10) and (11) below:

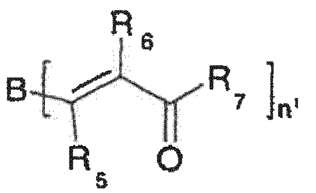
(10)

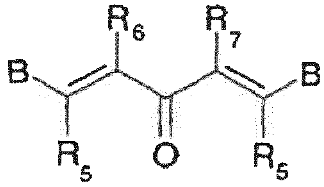

(11)

in which:

n' = 1 or 2,

B, in formula (10) when n' = 1 or in formula (11), is an aryl radical chosen from formulae (a') to (d') below, or in formula (10) when n' = 2, is a radical chosen from formulae (e') to (h') below:

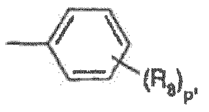

(a')

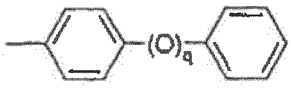

(b')

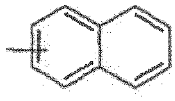

(c')

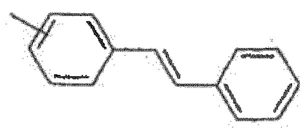

(d')

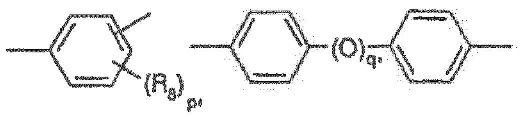

(e') (f')

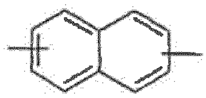

(g')

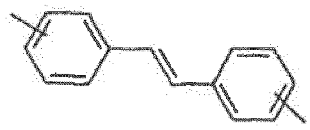

(h')

in which:

each of the symbols $R_8$ independently represents an OH group, a halogen atom, a linear or branched $C_{1-6}$ alkyl group optionally containing a silicon atom, a linear or branched $C_{1-6}$ alkoxy group optionally containing a silicon atom, a linear or branched $C_{1-5}$ alkoxycarbonyl group or a linear or branched $C_{1-6}$ alkylsulfonamide group optionally containing a silicon atom or an amino acid function,

p' represents an integer between 0 and 4 inclusive,

q' represents 0 or 1,

$R_5$ represents hydrogen or an OH group,

$R_6$ represents hydrogen, a linear or branched $C_{1-6}$ alkyl group optionally containing a silicon atom, a cyano group, a $C_{1-6}$ alkylsulfonyl group or a phenylsulfonyl group,

$R_7$ represents a linear or branched $C_{1-6}$ alkyl group optionally containing a silicon atom or a phenyl group possibly forming a bicycle and optionally substituted with one or two radicals $R_4$,

or $R_6$ and $R_7$ together form a monocyclic, bicyclic or tricyclic $C_{2-10}$ hydrocarbon-based residue, optionally interrupted with one or more nitrogen, sulfur and oxygen atoms and possibly containing another carbonyl, and optionally substituted with a linear or branched $C_1$-$C_8$ alkylsulfonamide group optionally containing a silicon atom or an amino acid function; on condition that when n' = 1, $R_6$ and $R_7$ do not form a camphor nucleus.

**8.** Composition according to Claim 1, in which the insoluble organic screening agents of the phenylenebis(benzoxazinone) type correspond to formula (12) below:

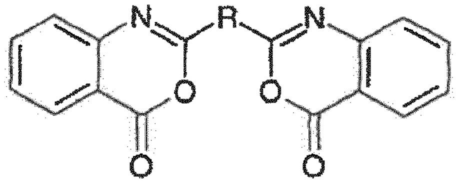

(12)

with R representing a divalent aromatic residue chosen from formulae (e") to (h") below:

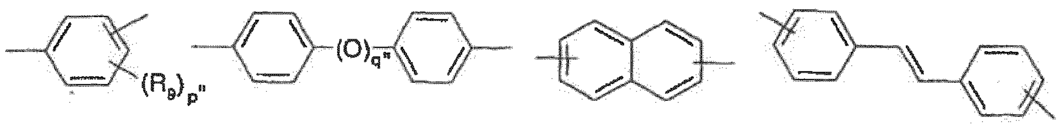

(e") (f") (g") (h")

in which:

each of the symbols $R_9$ independently represents an OH group, a halogen atom, a linear or branched $C_1$-$_6$ alkyl group optionally containing a silicon atom, a linear or branched $C_1$-$_6$ alkoxy group optionally containing a silicon atom, a linear or branched $C_1$-$_5$ alkoxycarbonyl group, or a linear or branched $C_1$-$_6$ alkylsulfonamide group optionally containing a silicon atom or an amino acid function,
p" represents an integer between 0 and 4 inclusive,
q" represents 0 or 1.

**9.** Composition according to Claim 1, in which the insoluble organic screening agents of the amide, sulfonamide or carbamate acrylonitrile derivative type are chosen from those corresponding to formula (13) below:

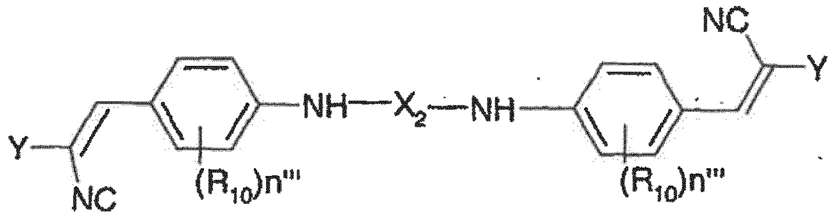

(13)

in which:

$R_{10}$ represents a linear or branched $C_1$-$_8$ alkyl group,
n''' is 0, 1 or 2,
$X_2$ represents a divalent radical of formula -(C=O)-$R_{11}$-(C=O)-, -$SO_2$-$R_{11}$-$SO_2$- or - (C=O)-O-$R_{11}$-O-(C=O)-, Y represents a radical -(C=O)-$R_{12}$ or -$SO_2R_{13}$,
$R_{11}$ represents a single bond or a linear or branched $C_1$-$C_{30}$ alkylene or $C_3$-$C_{30}$ alkenylene divalent radical, possibly bearing one or more hydroxyl substituents and possibly containing, in the carbon chain, one or more heteroatoms chosen from oxygen, nitrogen and silicon atoms,
$R_{12}$ represents a radical -$OR_{14}$ or -$NHR_{14}$,
$R_{13}$ represents a linear or branched $C_1$-$C_{30}$ alkyl radical or a phenyl nucleus, which is unsubstituted or substituted with $C_1$-$C_4$ alkyl or alkoxy radicals,
$R_{14}$ represents a linear or branched $C_1$-$C_{30}$ alkyl or $C_3$-$C_{30}$ alkenyl radical, possibly bearing one or more hydroxyl substituents and possibly containing, in the carbon chain, one or more heteroatoms chosen from oxygen, nitrogen and silicon atoms; and also isomers thereof.

**10.** Composition according to Claim 1, in which the insoluble UV-screening agents are chosen from multivalent metal salts of sulfonated benzylidene-camphor derivatives; multivalent metal salts of sulfonated benzimidazole derivatives; multivalent metal salts of cinnamic acid derivatives.

**11.** Composition according to any one of Claims 1 to 10, in which the compound(s) of formula (I) is (are) chosen from

those of formula (Ic) below:

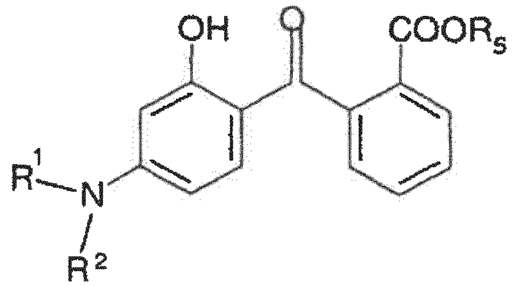

(Ic)

in which:

$R^1$ and $R^2$, which may be identical or different, denote a hydrogen atom, a $C_1$-$C_8$ alkyl radical or form, with the nitrogen atom to which they are attached, a 5- or 6-membered ring;
$R^5$ denotes a hydrogen atom, a $C_1$-$C_{12}$ alkyl radical or a $C_3$-$C_6$ cycloalkyl radical.

**12.** Composition according to Claim 11, in which the compound of formula (Ic) is n-hexyl 2-(4-diethylamino-2-hydroxybenzoyl)benzoate.

**13.** Composition according to any one of Claims 1 to 12, **characterized in that** it also comprises at least one agent for artificially tanning and/or browning the skin.

**14.** Composition according to any one of Claims 1 to 13, **characterized in that** it also comprises at least one soluble UV-A-active and/or UV-B-active organic UV-screening agent.

**15.** Composition according to any one of Claims 1 to 14, **characterized in that** it also comprises coated or uncoated metal oxide pigments.

**16.** Use of an amino-substituted 2-hydroxybenzophenone derivative of formula (I) as defined in the preceding claims, in a cosmetic composition for protecting the skin and/or the hair against ultraviolet radiation, in particular solar radiation, comprising at least one insoluble organic screening agent with a particle size ranging from 10 nm to 5 $\mu$m as defined in the preceding claims, for the purpose of producing a synergistic effect on the imparted UV-$A_{PPD}$ sun protection factors.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 1046391 A
- DE 10012408
- EP 1133980 A
- GB 2303549 A
- EP 893119 A
- WO 9522959 A
- WO 9703642 A
- GB 2286774 A
- EP 743309 A
- WO 9822447 A
- GB 2319523 A
- EP 0790243 A
- WO 9825922 A
- US 5888481 A
- DE 676103
- CH 350763
- US 5501850 A
- US 5961960 A
- EP 0669323 A
- US 5518713 A
- US 2463264 A
- EP 0921126 A
- EP 712855 A
- JP 04134042 B
- JP 04134043 B
- EP 0576974 A
- FR 2395023
- JP 01158090 A
- EP 0390683 A
- JP 04134041 B
- FR 2506156
- EP 0693471 A
- FR 2528420
- FR 2529887
- EP 0694521 A
- FR 2638354
- EP 0714880 A
- JP 04290882 B
- FR 2639347 A
- JP 87166517 B
- WO 9310753 A
- WO 9311095 A
- WO 9505150 A
- US 4367390 A
- EP 863145 A
- EP 517104 A
- EP 570838 A
- EP 796851 A
- EP 775698 A
- EP 878469 A
- EP 933376 A
- EP 669323 A
- US 5237071 A
- US 5166355 A
- DE 19726184
- WO 9304665 A
- DE 19855649
- EP 0967200 A
- DE 19746654
- DE 19755649
- EP 1008586 A
- EP 0518772 A
- EP 0518773 A
- FR 2315991
- FR 2416008

### Littérature non-brevet citée dans la description

- *J. Am. Chem. Soc.,* 1957, vol. 79, 5706-5708
- *J. Am. Chem. Soc.,* 1960, vol. 82, 609-611
- *J. Am.Chem. Soc.,* 1960, vol. 82, 609
- *J. Am. Chem. Soc.,* 1957, vol. 79, 5706-5708
- *J. Chim. Phys.,* 1967, vol. 64, 1602
- **E. MARIANI et al.** *16th IFSCC Congress,* 1990
- **BANGHAM ; STANDISH ; WATKINS.** *J. Mol. Biol.,* 1965, vol. 13, 238